# EUROPÄISCHE PATENTSCHRIFT

(11) **EP 1 723 118 B1**
(45) Veröffentlichungstag und Bekanntmachung des Hinweises auf die Patenterteilung: **08.07.2009**
(21) Anmeldenummer: 05715706.7
(22) Anmeldetag: 03.03.2005
(51) Int. Cl.: C07D 233/54, C07F 7/08, C07F 5/02, C07F 9/09

(54) **VERFAHREN ZUR HERSTELLUNG VON IONISCHEN VERBINDUNGEN, DEREN KATION EIN QUATERNÄRES, SP2-HYBRIDISIERTES STICKSTOFFATOM ENTHÄLT**
METHOD FOR THE PRODUCTION OF IONIC FLUIDS THE CATION OF WHICH COMPRISES A QUATERNARY SP2 - HYBRIDISED NITROGEN ATOM
PROCEDE DE PRODUCTION DE COMPOSES POURVUS D'ATOMES D'AZOTE QUATERNAIRES HYBRIDES SP2

(30) Priorität: 04.03.2004 DE 102004010662
(43) Veröffentlichungstag der Anmeldung: 22.11.2006
(73) Patentinhaber: BASF SE, 67056 Ludwigshafen (DE)
(72) Erfinder: SZARVAS, Laszlo, 67071 Ludwigshafen (DE); MAASE, Matthias, 67346 Speyer (DE); MASSONNE, Klemens, 67098 Bad Dürkheim (DE)
(74) Vertreter: Reitstötter - Kinzebach
(86) Internationale Anmeldenummer: PCT/EP2005/002253
(87) Internationale Veröffentlichungsnummer: WO 2005/085207

(56) Entgegenhaltungen:
- EP-A- 1 182 196
- EP-A- 1 182 197
- WO-A-01/81353
- WO-A-02/094883
- WASSERSCHEID P ET AL: "1-N-BUTYL-3-METHYLIMIDAZOLIUM (ÄBMIMÜ) OCTYLSULFATE-AN EVEN 'GREENER' IONIC LIQUID" GREEN CHEMISTRY, ROYAL SOCIETY OF CHEMISTRY, CAMBRIDGE,, GB, Bd. 4, Nr. 4, 2002, Seiten 400-404, XP008024341 ISSN: 1463-9262

## Beschreibung

Die vorliegende Erfindung betrifft ein Verfahren zur Herstellung von ionischen Verbindungen umfassend Kationen mit quaternären sp²-hybridisierten Stickstoffatomen, bei dem man Verbindungen, die ein doppelt gebundenes Stickstoffatom enthalten, mit einem Dialkylsulfat bei erhöhter Temperatur und unter Einsatz beider Alkylgruppen des Dialkylsulfats umsetzt und die so erhaltene ionische Verbindung mit Sulfatanionen gegebenenfalls einem Anionenaustausch unterzieht.

Salze, d. h. aus Kationen und Anionen aufgebaute heteropolare Verbindungen, zeichnen sich im Allgemeinen durch sehr hohe Schmelzpunkte aus, die in der Regel größer als 500 °C liegen. Klassische Schmelzen reiner Salze bzw. eutektischer Gemische besitzen zudem in der Regel eine hohe Viskosität. Ganz anders verhalten sich die so genannten ionischen Flüssigkeiten. Unter ionischen Flüssigkeiten versteht man Flüssigkeiten, die ausschließlich aus Ionen bestehen und die bei Raumtemperatur oder leicht erhöhter Temperatur (< 100 °C) flüssig sind. Derartige ionische Flüssigkeiten haben auf Grund ihrer speziellen Eigenschaften eine breite Verwendung gefunden. So dienen sie beispielsweise als Lösungsmittel für Übergangsmetall-katalysierte Reaktionen und als Extraktionsmittel zur Stofftrennung. Als so genannte "Designer Solvents" wird den ionischen Flüssigkeiten zukünftig ein großes Entwicklungspotential zugeschrieben, wobei davon ausgegangen wird, dass sich durch einen gezielten Austausch sowohl des kationischen als auch des anionischen Teils bis jetzt noch unbekannte, für den jeweiligen Zweck maßgeschneiderte Lösungsmittel darstellen lassen. Es besteht somit ein großer Bedarf an Verfahren, die sich zur Herstellung von ionischen Flüssigkeiten eignen.

J. S. Wilkes und M. J. Zaworotko beschreiben in J. Chem. Soc., Chem. Commun., 1992, S. 965 - 967 ionische Flüssigkeiten auf Basis des 1-Ethyl-3-methylimidazolium-Kations, die luft- und wasserstabil sind und sich daher für eine Vielzahl von Einsatzbereichen eignen. Ausgehend von der lodidverbindung lassen sich weitere Anionen, z. B. das Sulfat in Form seines Monohydrats, durch Anionenaustausch mit den entsprechenden Silbersalzen herstellen.

Der Einsatz von ionischen Verbindungen mit Halogenidanionen als Vorstufe für ionische Flüssigkeiten mit davon verschiedenen Anionen ist problematisch. So müssen ionische Flüssigkeiten speziell für einen Einsatz als Lösungsmittel in der Übergangsmetalikatalyse spezielle Reinheitsanforderungen erfüllen. Für viele Übergangsmetallkatalysatoren wirken Spuren von Halogenidionen, insbesondere Chlorid, Bromid und lodid, als Katalysatorgifte. Auf Grund ihrer nicht flüchtigen Natur lassen sich anorganische Flüssigkeiten nicht wie organische Lösungsmittel destillativ reinigen. Ein selektiver Austausch von Halogenidionen gegen andere Anionen bzw. eine selektive Entfernung von Restmengen enthaltener Halogenidionen aus ionischen Flüssigkeiten ist zwar durch Einsatz von Anionenaustauschern möglich, durch die hohen entstehenden Kosten jedoch wirtschaftlich unattraktiv. Es ist daher eine bereits halogenidfreie Synthese der ionischen Flüssigkeiten anzustreben.

Die EP-A-1 182 196 beschreibt ein Verfahren zur Herstellung ionischer Flüssigkeiten, bei dem man die dem Kation zu Grunde liegenden Amine, Phosphine, Imidazole, Pyridine, Triazole oder Pyrazole mit einem Dialkylsulfat alkyliert, wobei Salze der entsprechenden Monoalkylsulfatanionen erhalten werden und man diese anschließend einem Anionenaustausch mit Metallsalzen unterzieht.

Die WO 00/32658 beschreibt ionische Flüssigkeiten und deren Einsatz zur Herstellung von Polyisoolefinen mit hohem Molekulargewicht.

Die WO 03/074494 beschreibt halogenfreie ionische Flüssigkeiten auf Basis von Anionen der Formeln [R'-O-SO₃]⁻ oder [R'-SO₃]⁻, wobei R' eine Gruppe der allgemeinen Formel R⁵-[X(-CH₂-)ₙ]ₘ darstellt, in der n eine Zahl zwischen 1 und 12 ist, m eine Zahl zwischen 1 und 400 ist, X für Sauerstoff, Schwefel oder eine Gruppe der allgemeinen Formeln -O-Si(CH₃)₂-O-, -O-Si(CH₂CH₃)₂-O-, -O-Si(OCH₃)₂-O- oder -O-Si(O-CH₂CH₃)₂-O- steht und R⁵ eine lineare oder verzweigte, gesättigte oder ungesättigte, aliphatische oder alicyclische, unfunktionalisierte oder miteiner oder mehreren Gruppen Y funktionalisierte Alkylgruppe mit 1 bis 36 Kohlenstoffatomen darstellt, wobei Y eine -OH, -OR", -COOH, -COOR", -NH₂, -SO₄, -F, -Cl, -Br, -I oder-CH-Gruppe ist und dabei R" eine verzweigte oder lineare Kohlenwasserstoffkette mit 1 bis 12 Kohlenstoffatomen repräsentiert. Ihre Herstellung erfolgt ausgehend von Pyridin-SO₃-Komplexen und Ethern der Formel R'-OH.

Es ist bekannt, dass sich Amine mit Dialkylsulfaten alkylieren lassen, wobei in der Regel jedoch nur eine Alkylgruppe des Dialkylsulfats ausgenutzt wird, so dass die entsprechenden Monoalkylsulfatsalze resultieren. Die DE-OS-15 43 747 beschreibt ein Verfahren zur direkten Herstellung, eines bisquatemären Ammoniumsalzes aus einem Dialkylsulfatester und einem Trialkylamin durch Umsetzung bei einer Temperatur im Bereich von 0 bis 400 °C und einem ausreichenden Druck, um die Verdampfung des Amins zu verhindern. Da bei erhöhten Temperaturen eine Hydrolyse des Sulfatesters stattfindet, lehrt dieses Dokument die Umsetzung zweistufig durchzuführen, wobei zunächst bei einer niedrigen Temperatur im Bereich von etwa 0 bis 50 °C eine Alkylgruppe des Sulfatesters und dann in einem zweiten Schritt bei einer erhöhten Temperatur im Bereich von etwa 50 bis 400 °C die zweite Alkylgruppe zur Alkylierung eingesetzt wird.

Der vorliegenden Erfindung liegt die Aufgabe zu Grunde, ein einfaches und somit wirtschaftliches Verfahren zur Herstellung von ionischen Verbindungen zur Verfügung zu stellen, die sich als oder zur Herstellung von ionischen Flüssigkeiten eignen. Insbesondere soll sich das Verfahren zur Herstellung von ionischen Flüssigkeiten eignen, die im Wesentlichen frei von Halogeniden, speziell frei von Chlorid, Bromid und lodid, sind.

Überraschenderweise wurde nun gefunden, dass diese Aufgabe durch ein Verfahren gelöst wird, bei dem man eine Verbindung, die ein doppelt gebundenes (sp²-hybridisiertes) Stickstoffatom enthält, mit einem Dialkylsulfat bei erhöhten Temperaturen umsetzt, wobei eine ionische Verbindung erhalten wird, die als Anionkomponente ein Sulfatanion enthält, und gegebenenfalls anschließend das Sulfatanion gegen ein davon verschiedenes Anion austauscht.

Gegenstand der Erfindung ist daher Verfahren zur Herstellung einer ionischen Verbindung, die wenigstens ein Kation mit einem quaternären sp²-hybridisierten Stickstoffatom umfasst, bei dem man
a) eine Verbindung, die ein doppelt gebundenes Stickstoffatom enthält, mit einem Dialkylsulfat unter Einsatz beider Alkylgruppen des Dialkylsulfats umsetzt, wobei eine ionische Verbindung mit Sulfatanionen erhalten wird, und
b) die in Schritt a) erhaltene ionische Verbindung gegebenenfalls einem Anionenaustausch unterzieht.

Überraschenderweise wurde gefunden, dass Verbindungen, die wenigstens ein doppelt gebundenes Stickstoffatom aufweisen, sich mit Dialkylsulfaten unter Einsatz beider Alkylgruppen quatemisieren lassen. Vorteilhafterweise werden somit ionische Verbindungen erhalten, die als Anionenkomponente zweifach negativ geladene Sulfatanionen anstelle von einfach negativ geladenen Alkylsulfatanionen aufweisen. Somit können zum einen die Alkylgruppenäquivalente des Dialkylsulfats effektiv ausgenutzt werden, zum anderen sind die erhaltenen Sulfatverbindungen gute Zwischenprodukte für die Herstellung halogenidfreier ionischer Flüssigkeiten. Vorteilhafterweise wird die im Stand der Technik als Nachteil der zweifachen Alkylierung mit Dialkylsulfaten beschriebene Hydrolyse beim erfindungsgemäßen Verfahren nicht beobachtet. Dass sich Verbindungen, die wenigstens ein doppelt gebundenes Stickstoffatom aufweisen, zur erfindungsgemäßen Alkylierung unter Einsatz beider Alkylgruppen eines Dialkylsulfats eignen, ist insoweit überraschend, als in diesen Verbindungen das Stickstoffatom sp²-hybridisiert ist und diese Verbindungen schwächere Basen sind als Amine, in denen das Stickstoffatom sp³-hybridisiert vorliegt.

Unter Verbindungen mit wenigstens einem doppelt gebundenen Stickstoffatom werden im Rahmen der vorliegenden Erfindung auch Resonanz-stabilisierte Verbindungen, z. B. aromatische Verbindungen, verstanden, in denen nur einzelne Resonanzstrukturen (Mesomerengrenzstrukturen) eine Doppelbindung zum Stickstoffatom aufweisen.

Für den Zweck der Erläuterung der vorliegenden Erfindung umfasst der Ausdruck "Alkyl" geradkettige, und verzweigte Alkylgruppen. Vorzugsweise handelt es sich dabei um geradkettige oder verzweigte C₁-C₂₀-Alkyl, bevorzugterweise C₁-C₁₀-Alkyl-, besonders bevorzugt C₁-C₈-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1;2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-0ctyl, 2-Ethylhexyl, 2-Propylheptyl, Nonyl, Decyl.

Der Ausdruck "Alkyl" umfasst auch substituierte Alkylgruppen, welche im Allgemeinen 1, 2, 3, 4 oder 5, bevorzugt 1, 2 oder 3 und besonders bevorzugt 1 Substituenten aufweisen. Diese sind beispielsweise ausgewählt unter Cycloalkyl, Aryl, Hetaryl, Halogen, Amino, Alkoxycarbonyl, Acyl, Nitro, Aminocarbonyl, Alkylaminocarbonyl, Dialkylaminocarbonyl, Alkylcarbonylamino, Carboxylat und Sulfonat.

Der Ausdruck "Alkylen" im Sinne der vorliegenden Erfindung steht für geradkettige oder verzweigte Alkandiyl-Gruppen mit vorzugsweise 1 bis 5 Kohlenstoffatomen.

Der Ausdruck "Cycloalkyl" umfasst im Sinne der vorliegenden Erfindung unsubstituierte als auch substituierte Cycloalkylgruppen, vorzugsweise C₅-C₈-Cycloalkylgruppen, wie Cyclopentyl, Cyclohexyl oder Cycloheptyl. Diese können im Falle einer Substitution, im Allgemeinen 1, 2, 3, 4 oder 5, bevorzugt 1, 2 oder 3 Substituenten tragen. Diese Substituenten sind beispielsweise ausgewählt unter Alkyl und den zuvor für substituierte Alkylgruppen genannten Substituenten.

Der Ausdruck "Heterocycloalkyl" im Sinne der vorliegenden Erfindung umfasst gesättigte, cycloaliphatische Gruppen mit im Allgemeinen 4 bis 7, vorzugsweise 5 oder 6 Ring-atomen, in denen 1, 2, 3 oder 4 der Ringkohlenstoffatome durch Heteroatome, ausgewählt aus den Elementen Sauerstoff, Stickstoff und Schwefel, ersetzt sind und die gegebenenfalls substituiert sein können. Im Falle einer Substitution können diese heterocycloaliphatischen Gruppen z. B. 1, 2 oder 3 Substituenten tragen. Diese Substituenten sind beispielsweise ausgewählt unter Alkyl und den zuvor für substituierte Alkylgruppen genannten Substituenten. Beispielhaft für solche heterocycloaliphatischen, Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl, Tetrahydrothiophenyl, Tetrahydrofuranyl, Tetrahydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl. Diese Arylgruppen können im Falle einer Substitution im Allgemeinen 1, 2, 3, 4 oder 5, vorzugsweise 1, 2 oder 3 Substituenten tragen. Diese Substituenten sind beispielsweise ausgewählt unter Alkyl und den zuvor für substituierte Alkylgruppen genannten Substituenten.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl. Diese heterocycloaromatischen Gruppen können im Falle einer Substitution im Allgemeinen 1, 2 oder 3 Substituenten. Diese Substituenten sind beispielsweise ausgewählt unter Alkyl und den zuvor für substituierte Alkylgruppen genannten Substituenten.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäureester- oder S ulfonsäureesterfunktion oder eine Carbonsäure- oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Gruppen NE¹E²stehen vorzugsweise für N,N-Dimethylamino, N,N-Diethylamino, N,N-Dipropylamino, N,N-Diisopropylamino, N,N-Di-n-butylamino. N,N-Di-t.-butylamino, N,N-Dicyclohexylamino oder N,N-Diphenylamino.

Halogen steht für Fluor, Chlor, Brom und lod, bevorzugt für Fluor, Chlor und Brom.

M⁺ steht für ein Kationäquivalent, d. h. für ein einwertiges Kation oder den einer positiven Einfachladung entsprechenden Anteil eines mehrwertigen Kations. Das Kation M⁺ dient lediglich als Gegenion zur Neutralisation negativ geladener Substituentengruppen, wie der COO' oder der Sulfonatgruppe und kann im Prinzip beliebig gewählt werden. Vorzugsweise werden deshalb Alkalimetall-, insbesondere Na⁺, K⁺-, Li⁺-tonen oder Onium-lonen, wie Ammonium-, Mono-, Di-, Tri-, Tetraalkylammonium-, Phosphonium-, Tetraalkylphosphonium- oder Tetraarylphosphonium-lonen verwendet.

Entsprechendes gilt für das Anionäquivalent A⁻, das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions, wobei im Allg emeinen von Halogenid-lonen verschiedene Anionen bevorzugt sind.

Das erfindungsgemässe Verfahren eignet sich ganz allgemein zur Herstellung von ionischen Verbindungen der Formel I

bB^{m+}xXⁿ⁻ (I)

worin
- B^{m+}: für ein m-wertiges Kation mit wenigstens einem quaternären sp²-hybridisierten Stickstoffatom steht,
- Xⁿ⁻: für ein n-wertlges Anion steht,

b und x für ganze Zahlen ≥ 1 stehen, mit der Massgabe, das (b mal m) = (x mal n) ist.

Dazu zählen Verbindungen der Formeln B⁺ X⁻, B^{m+} X^{m-}, nB⁺ Xⁿ⁻ und S^{m+} mX⁻, worin m und n für ganze Zahlen > 1 stehen.

Vorzugsweise handelt-es sich bei der Anionkomponente Xⁿ⁻ um ein von Cl⁻, Br⁻, I⁻ und Monoalkylsulfaten verschiedenes Anion. Vorzugsweise sind die Anionen Xⁿ⁻ ausgewählt unter Sulfat (SO₄²⁻), Hydrogensulfat (HSO₄⁻), Nitrit (NO₂⁻), Nitrat (NO₃⁻), Cyanid (CN⁻), Cyanat (OCN⁻), Isocyanat (NCO⁻), Thiocyanat (SCN⁻), Isothiocyanat (NCS⁻), Phosphat (PO₄³⁻), Hydrogenphosphat (HPO₄²⁻), Dihydrogenphosphat (H₂PO₄⁻), primärem Phosphit (H₂PO₃⁻), sekundärem Phosphit (HPO₃²⁻), Orthoborat (BO₃³⁻), Metaborat ((BO₂)₃³⁻), Tetrafluoroborat ([BF₄]⁻), Tetrachloroborat ([BCl₄]⁻), Tetraphenylborat ([B(C₆H₅)₄]⁻), Hexafluorophosphat ([PF₆]⁻, Hexafluoroantimonat ([SbF₆]⁻), Hexafluoroarsenat ([AsF₆]⁻], Tetrachloroaluminat ([AlCl₄]⁻), Tetrachbromoaluminat ([AlBr₄]⁻), Trichlorozinkat ([ZnCl₃]⁻), Dichlorocupraten(I) und (II), Carbonat (CO₃²⁻), Hydrogencarbonat (HCO₃⁻), Fluorid (F⁻), Triorganylsilanolat R'₃SiO⁻ Fluorosulfonat (CF₃-SO₃)⁻, Sulfonat (R'-SO₃)⁻ und [(R'-SO₂)₂N]⁻, worin R' für Alkyl, Cycloalkyl oder Aryl steht. Bevorzugt ist R ein linearer oder verzweigter 1 bis 12 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest, der durch Halogenatome substituiert sein kann.

Besonders bevorzugt ist Xⁿ⁻ ausgewählt unter SO₄²⁻ und Anionen organischer Monocarbonsäuren, vorzugsweise Acetat.

Bei dem Kation kann es sich um eine acyclische oder cyclische Verbindung handeln. Vorzugsweise leitet sich das Kation mit wenigstens einem quaternären sp²-hybridisierten Stickstoffatom ab von Iminen, Diazenen (Azo-Verbindungen), Amidinen, Amidoximen, Amidrazonen, Oximen, Sulfimiden, Guanidinen, Phosphiniminen, Stickstoff-haltigen aromatischen Heterocyclen, etc.

Bevorzugt leitet sich das Kation von einer aliphatischen Guanidin-Verbindung ab.

Desweiteren bevorzugt leitet sich das Kation von einer heterocycloaliphatischen Verbindung ab, die vorzugsweise ausgewählt ist unter 4,5-Dihydropyrazolen, 4,5-Dihydrooxazolen, 4,5-Dihydrothiazolen und 2-Imidazolinen.

Desweiteren bevorzugt leitet sich das Kation von einer heterocycloaromatischen Verbindung ab, die vorzugsweise ausgewählt ist unter Pyrrolen, Imidazolen, Pyrazolen, Indolen, Carbazolen, Benzimidazolen, Indazolen, Purinen, 1,2,3-Triazolen, 1,2,4-Triazolen, Benzotriazolen, Oxazolen, Isoxazolen, Thiazolen, Isothiazolen, Pyridinen, Pyridazinen, Pyrimidinen, Pyrazinen, Chinolinen, Isochinolinen, Phenanthridinen, Cinnolinen, Chinazolinen, Phthalazinen, Chinoxalinen, 1,8-Naphthyridinen, Pteridinen, Phenazinen, Acridinen, 1,3,5-Triazinen, 1,2,4-Triazinen, Benzotriazinen und Tetrazinen.

Überraschenderweise wurde weiterhin gefunden, dass Verbindungen, die wenigstens ein doppelt gebundenes Stickstoffatom aufweisen und die zusätzlich befähigt sind, eine positive Ladung an diesem Stickstoffatom zu delokalisieren, sich besonders vorteilhaft mit Dialkylsulfaten unter Einsatz beider Alkylgruppen quatemisieren lassen.

Bevorzugt ist daher ein Verfahren zur Herstellung von Verbindungen der allgemeinen Formel II 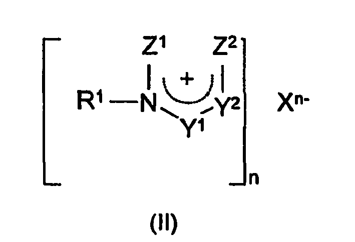 worin
- R¹: für C₁- bis C₁₀-Alkyl steht,
- Y¹ und Y²: unabhängig voneinander ausgewählt sind unter Heteroatomen und heteroatomhaltigen Gruppe, die jeweils ein freies Elektronenpaar aufweisen, sowie Gruppen CR², worin das Kohlenstoffatom sp²-hybridisiert ist und R² für Wasserstoff oder einen Organylrest steht,
- Z¹ und Z²: unabhängig voneinander für einfach oder doppelt gebundene Organylreste stehen, wobei Z¹ und Z² auch gemeinsam für eine verbrückende Gruppe mit 2 bis 5 Atomen zwischen den flankierenden Bindungen stehen können,
- Xⁿ⁻: für ein Anion steht, das vorzugsweise von Cl⁻, Br⁻, I⁻ und Monoalkylsulfat verschieden ist, und
- n: für eine ganze Zahl von 1 bis 3 steht,
wobei die Gruppe NR¹-Y¹-Y² und gegebenenfalls zusätzlich Z¹ und/oder Z² Teil eines delokalisierten π-Elektronensystems sind,
bei dem man
a) eine Verbindung der allgemeinen Formel 11.1 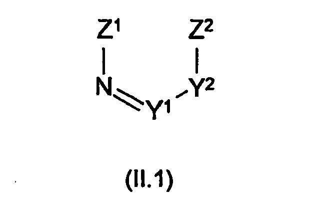 worin Y¹, Y², Z¹ und Z² die angegebenen Bedeutungen besitzen, mit einem Dialkylsulfat (R¹)₂SO₄, worin R¹ für C₁- bis C₁₀-Alkyl steht, bei erhöhter Temperatur und unter Einsatz beider Alkylgruppen des Dialkylsulfats zu einer Verbindung der Formel II umsetzt, worin Xⁿ⁻ für ein Sulfatanion steht, und
b) gegebenenfalls das Sulfatanion gegen ein davon verschiedenes Anion austauscht.

Die Kationen der Verbindungen der allgemeinen Formel II sind zu einer Delokalisierung der positiven Ladung vom alkylierten Stickstoffatom über wenigstens einen Teil des restlichen Moleküls befähigt.

Bevorzugt steht in den Verbindungen der Formeln II und II.1 der Rest R¹ für C₁-C₄-Alkyl und insbesondere für Methyl oder Ethyl.

Die Werte für y stehen für eine ganze Zahl von 1 bis 240, vorzugsweise für eine ganze Zahl von 3 bis 120.

Vorzugsweise sind in den Verbindungen der Formeln II und 11.1 die Gruppen Y¹ und Y² unabhängig voneinander ausgewählt unter O, S, CR², NR³ oder PR⁴ worin R², R³ und R⁴ unabhängig voneinander für Wasserstoff, Alkyl, Cycloalkyl, Heterocycloalkyl, Aryl, Hetaryl, COOR^{a}, COO⁻M⁺, SO₃R^{a}, SO₃⁻M⁺, Sulfonamid, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, Alkylaminocarbonyl, Di alkylaminocarbonyl, Alkylcarbonylamino, Halogen, Nitro, Acyl oder Cyano stehen, worin
- R^{a}: jeweils gleiche oder verschiedene Reste, ausgewählt unter Wasserstoff, Alkyl, Cycloalkyl, Aryl, Heterocycloalkyl oder Hetaryl bedeuten,
- E¹, E², E³: jeweils gleiche oder verschiedene Reste, ausgewählt unter Wasserstoff, Alkyl, Cycloalkyl, Aryl oder Hetaryl bedeuten,
- R^{b}: für Wasserstoff, Methyl oder Ethyl steht,
- M⁺: für ein Kationäquivalent steht,
- A⁻: für ein Anionäquivalent steht und
- y: für eine ganze Zahl von 1 bis 250 steht.

In einer bevorzugten Ausführung steht eine der Gruppen Y¹ oder Y² für eine Gruppe der Formel CR² und die andere ist ausgewählt unter O, S, CR², NR³ oder PR⁴, Besonders bevorzugt steht dann eine der Gruppen Y¹ oder Y² für eine Gruppe der Formel CR² und die andere ist ausgewählt unter O, S, oder NR³, Speziell steht die Gruppe Y¹ für eine Gruppe der Formel CR².

Bevorzugt steht R² für Wasserstoff. Des Weiteren bevorzugt steht R² für eine Gruppe der Formel OR^{c}, SR^{c} oder NR^{c}R^{d}, worin R^{c} und R^{d} jeweils gleiche oder verschiedene Reste, ausgewählt unter Wasserstoff, Alkyl, Cycloalkyl, Heterocycloalkyl, Aryl oder Hetaryl bedeuten. Solche Gruppen R² ermöglichen die positive Ladung des Kations der Verbindungen I zusätzlich durch Delokalisierung zu stabilisieren.

Bevorzugt sind R³ und R⁴ ausgewählt unter Wasserstoff, Alkyl, vorzugsweise C₁-C₁₀-Alkyl, wie Methyl, Ethyl, Propyl, Isopropyl, n-Butyl und tert.-Butyl, Cycloalkyl, vorzugsweise C₅-C₇-Cycloalkyl, wie Cyclopentyl und Cyclohexyl und Aryl, insbesondere Phenyl.

Nach einer ersten bevorzugten Ausführungsform sind Z¹ und Z² nicht miteinander verbrückt. Dann sind Z¹ und Z² vorzugsweise unabhängig voneinander ausgewählt unter Alkyl, Cycloalkyl, Heterocycloalkyl, Aryl und Hetaryl, wie eingangs definiert.

Wenn Z¹ und Z² nicht miteinander verbrückt sind, handelt es sich bei den Kationen der Verbindungen der Formel II vorzugsweise um ein Guanidiniumion der Formel 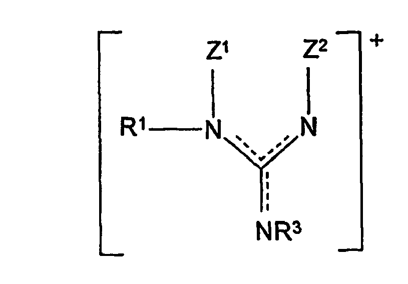 worin R¹, R³, Z¹ und Z² die zuvor angegebenen Bedeutungen besitzen.

In einer bevorzugten Ausführungsform sind Z¹ und Z² miteinander verbrückt. Dann stehen Z¹ und Z² gemeinsam mit der Gruppe NR¹+Y¹-Y², an die sie gebunden sind, vorzugsweise für einen 5- bis 8-gliedrigen Heterocyclus, der gegebenenfalls ein-, zwei- oder dreifach mit Cycloalkyl, Heterocycloalkyl, Aryl und/oder Hetaryl anelliert ist, wobei die anellierten Gruppen unsubstituiert sind oder unabhängig voneinander je einen, zwei, drei oder vier Substituenten tragen können. Die Substituenten der anellierten Gruppen sind vorzugsweise ausgewählt unter Alkyl, Alkoxy, Amino, Polyalkylenoxid, Polyalkylenimin, Halogen, Nitro, Cyano, Sulfonat und Carboxylat.

Vorzugsweise stehen Z¹ und Z² gemeinsam für eine verbrückende Gruppe mit zwei bis drei Atomen zwischen den flankierenden Bindungen, die ausgewählt sind unter gegebenenfalls substituierten Heteroatomen und sp²-hybridisierten Kohlenstoffatomen, wobei die verbrückende Gruppe gemeinsam mit der Gruppe NR¹-Y¹-Y² ein delokalisiertes π-Elektronensystem bildet.

Bevorzugt leitet sich das Kation der Verbindungen der Formel I von einer heterocycloaromatischen Gruppe ab, die vorzugsweise ausgewählt ist unter Pyrrol-, Imidazol-, Pyrazol-, 1,2,3-Triazol-, 1,2,4-Triazol-, Indol-, Oxazol-, Thiazol-, Pyridin-, Pyrimidin-, 1,3,5-Triazin- und 1,2,4-Triazin-Gruppen.

Besonders bevorzugt ist die Verbindung der Formel II ausgewählt unter Verbindungen der Formel II.a bis II.e 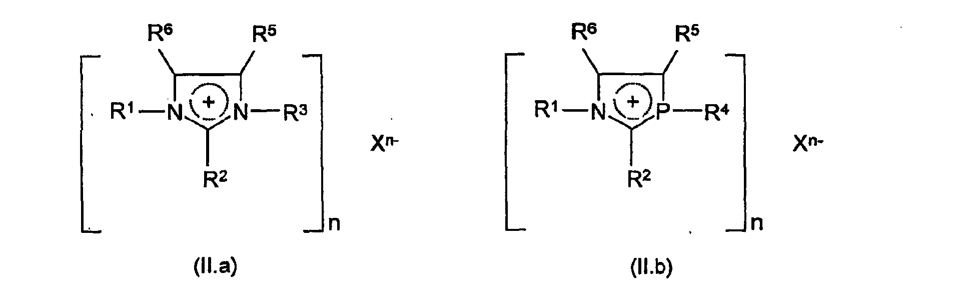 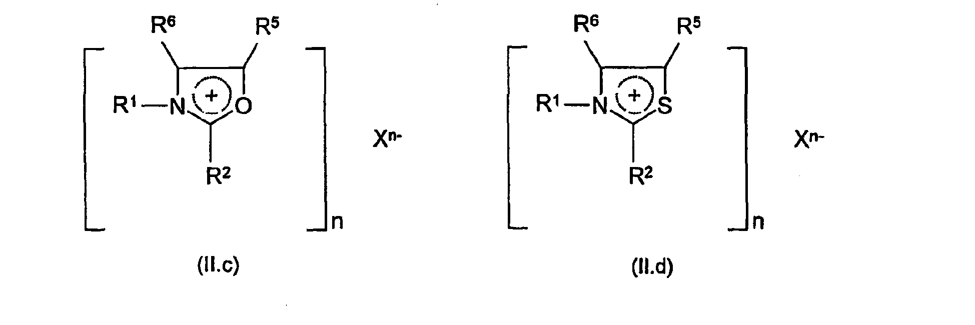 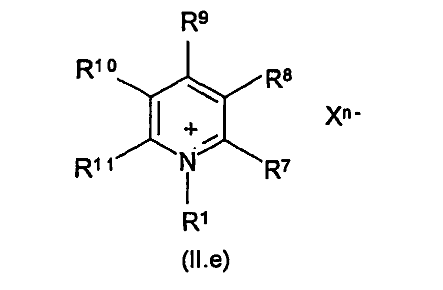 worin
- Xⁿ⁻: für ein Anion steht, das vorzugsweise von Cl⁻, Br⁻, I⁻ und Monoalkylsulfat verschieden ist, und,
- n: für eine ganze Zahl von 1 bis 3 steht,
- R¹: für C₁- bis C₁₀-Alkyl steht, und
- R²,: R³, R⁴, R⁵, R⁶, R⁷, R⁸, R⁹, R¹⁰ und R¹¹ unabhängig voneinander für Wasserstoff, Alkyl, Cycloalkyl, Heterocycloalkyl, Aryl, Hetaryl, COOR^{a}, COO⁻M⁺, SO₃R^{a}, SO₃⁻ M⁺, Sulfonamid, NE¹E², (NE¹E²E³)⁺A⁻, OR^{a}, SR^{a}, (CHR^{b}CH₂O)_{y}R^{a}, (CH₂O)yR^{a}, (CH₂CH₂NE¹)_{y}R^{a}, Alkylaminocarbonyl, Dialkylaminocarbonyl, Alkylcarbonylamino, Halogen, Nitro, Acyl oder Cyano stehen, wobei
- R^{a}: jeweils gleiche oder verschiedene Reste, ausgewählt unter Wasserstoff, Alkyl, Cycloalkyl, Aryl oder Hetaryl bedeuten,
- E¹, E², E³: jeweils gleiche oder verschiedene Reste, ausgewählt unter Wasserstoff, Alkyl, Cycloalkyl, Aryl oder Hetaryl bedeuten,
- R^{b}: für Wasserstoff, Methyl oder Ethyl steht,
- M⁺: für ein Kationäquivalent steht,
- A⁻: für ein Anionäquivalent steht und
- y: für eine ganze Zahl von 1 bis 250 steht.

Bevorzugt stehen bei den Verbindungen der Formeln II.a bis II.d die Reste R², R⁵ und R⁶ unabhängig voneinander für Wasserstoff oder Alkyl, insbesondere C₁-C₆-Alkyl, wie Methyl, Ethyl, Propyl, Isopropyl, n-Butyl und tert.-Butyl.

Bevorzugt stehen bei den Verbindungen der Formel II.e die Reste R⁷, R⁸, R⁹, R¹⁰ und R¹¹ unabhängig voneinander für Wasserstoff oder Alkyl, insbesondere C₁-C₆-Alkyl, wie Methyl, Ethyl, Propyl, Isopropyl, n-Butyl und tert.-Butyl. Bevorzugt steht einer der Reste R⁷, R⁸, R⁹, R¹⁰ und R¹¹ für Alkyl, speziell für Methyl, und die übrigen stehen für Wasserstoff.

Zur erfindungsgemäßen Herstellung von ionischen Verbindungen, die wenigstens ein Kation mit einem quaternären sp²-hybridisierten Stickstoffatom umfassen, wird in einem ersten Reaktionsschritt a) eine Verbindung, die ein doppelt gebundenes Stickstoffatom enthält, mit einem Dialkylsulfat unter Einsatz beider Alkylgruppen des Dialkylsulfats umgesetzt, wobei eine ionische Verbindung mit Sulfatanionen erhalten wird, und gegebenenfalls anschliessend in einem Schritt b) die in Schritt a) erhaltene ionische Verbindung einem Anionenaustausch unterzogen.

Erfindungsgemäß erfolgt die Umsetzung in Schritt a) bei einer erhöhten Temperatur, d.h. bei einer Temperatur, die oberhalb der Umgebungstemperatur liegt. Vorzugsweise beträgt die Temperatur in Schritt a) wenigstens 60 °C, besonders bevorzugt wenigstens 80 °C. Vorzugsweise erfolgt die Umsetzung in Schritt a) bei einer Temperatur im Bereich von 100 bis 220 °C, besonders bevorzugt von 120 bis 200 °C.

Die Umsetzung in Schritt a) kann unter Umgebungsdruck sowie unter vermindertem und erhöhtem Druck erfolgen. Bevorzugt erfolgt die Reaktion unter dem Eigendruck der Reaktionsmischung bei den Reaktionsbedingungen. Beim Einsatz flüchtiger Amine beträgt der Druck bei der Umsetzung in Schritt a) im Allgemeinen mindestens 1,5 bar; insbesondere mindestens 2 bar. Gewünschtenfalls kann der Druck bei der Umsetzung in Schritt a) bis 300 bar betragen. Geeignete druckfeste Reaktoren sind dem Fachmann bekannt und werden z. B. in Ullmanns Enzyklopädie der technischen Chemie, Band 1, 3. Auflage, 1951, S. 769 ff beschrieben. Im Allgemeinen wird für das erfindungsgemäße Verfahren ein Autoklav verwendet, der gewünschtenfalls mit einer Rührvorrichtung und/oder einer Innenauskleidung versehen sein kann.

Das Molmengenverhältnis von von der zu alkylierenden Verbindung, die ein doppelt gebundenes Stickstoffatom enthält, zu dem Dialkylsulfat beträgt vorzugsweise wenigstens 2:1. Besonders bevorzugt liegt das Molmengenverhältnis von der zu alkylierenden Verbindung zu dem Dialkylsulfat in einem Bereich von 1,8:1 bis 10:1, insbesondere 2,05:1 bis 5:1, speziell 2,1:1 bis 3:1.

Die Umsetzung der zu alkylierenden Verbindung mit dem Dialkylsulfat kann in Substanz oder in Gegenwart eines unter den Reaktionsbedingungen inerten Lösungsmittels erfolgen. Geeignete Lösungsmittel sind z. B. Wasser, wassermischbare Lösungsmittel, beispielsweise Alkohole, wie Methanol und Ethanol, und Mischungen davon. Bevorzugt wird als Lösungsmittel Wasser oder ein Lösungsmittelgemisch eingesetzt, das mindestens 30 Vol-%, bevorzugt mindestens 50 Vol-%, insbesondere mindestens 80 Vol-% Wasser umfasst.

Bei den in Schritt a) eingesetzten Dialkylsulfaten handelt es sich vorzugsweise um Di-C₁-C₁₀-alkylsulfate und insbesondere um Di-C₁-C₆-alkylsulfate, wie Dimethyl-, Diethyl-, Di-n-propyl-, Diisopropyl-, Di-n-butyl-, Diisobutyl-, Di-tert.-butyl-, Di-n-pentyl-, Diisopentyl-, Dineopentyl- und Di-n-hexylsulfat. Besonders bevorzugt werden Dimethylsulfat und Diethylsulfat eingesetzt.

Gewünschtenfalls kann die Umsetzung in Schritt a) in Gegenwart wenigstens eines Inertgases erfolgen. Geeignete Inertgase sind beispielsweise Stickstoff, Helium und Argon.

Die Umsetzung in Schritt a) kann kontinuierlich oder diskontinuierlich erfolgen.

Aus dem in Schritt a) erhaltenen Reaktionsgemisch kann das Sulfatsalz nach üblichen, dem Fachmann bekannten Verfahren isoliert werden. Wurde zur Umsetzung in Schritt a) ein Lösungsmittel eingesetzt, so kann dieses durch Verdampfen, vorzugsweise unter verringertem Druck, entfernt werden. Da die erhaltenen ionischen Verbindungen nicht flüchtig sind, ist der eingesetzte Druckbereich in der Regel nicht kritisch. Sofern eine möglichst vollständige Entfernung des Lösungsmittels gewünscht ist, kann beispielsweise ein Feinvakuum von 10¹ bis 10⁻¹ Pa oder ein Hochvakuum von 10⁻¹ bis 10⁻⁵ Pa eingesetzt werden. Zur Druckerzeugung können übliche Vakuumpumpen, wie Flüssigkeitsstrahlvakuumpumpen, Dreh- und Sperrschiebervakuumpumpen, Membranvakuumpumpen, Diffusionspumpen etc. eingesetzt werden. Das Entfernen des Lösungsmittels kann zudem bei einer erhöhten Temperatur von bis zu 150 °C, bevorzugt bis zu 100 °C erfolgen.

Sofern sich an die Alkylierungsreaktion in Schritt a) eine Anionenaustauschreaktion in Schritt b) anschließt, kann das in Schritt a) erhaltene Sulfatsalz nach den zuvor beschriebenen Verfahren isoliert werden. Dies gilt speziell, wenn die Umsetzung in Schritt b) in einem anderen Lösungsmittel erfolgen soll als die Alkylierung in Schritt a). In einer weiteren Ausführung wird zur Umsetzung in Schritt b) das in Schritt a) erhaltene Reaktionsgemisch ohne vorherige Isolierung eingesetzt.

Der Austausch des Sulfatanions in Schritt b) kann durch Umprotonierung mit H₂SO₄, Umsetzung mit einem Metallsalz, Ionenaustauschchromatographie oder eine Kombination dieser Maßnahmen erfolgen.

In einer ersten Ausführungsform wird die in Schritt a) des erfindungsgemässen Verfahrens erhaltene ionische Verbindung auf Basis von Sulfatanionen mit Schwefelsäure unter Protonenübertragung umgesetzt, wobei die entsprechenden Hydrogensulfate erhalten werden (Xⁿ⁻ = HSO₄⁻). Vorzugsweise erfolgt die Umprotonierung mit 100%iger H₂SO₄. Das Molmengenverhältnis von H₂SO₄ zu SO₄²⁻ beträgt vorzugsweise ≥1 : 1 und liegt beispielsweise in einem Bereich von 1:1 bis 2:1. Die dabei resultierenden ionischen Verbindungen auf Basis von Hydrogensulfatanionen eignen sich in der Regel sowohl als ionische Flüssigkeiten, wie auch als Zwischenprodukte für einen weiteren Anionenaustausch.

In einer weiteren Ausführungsform erfolgt der Anionenaustausch in Schritt b) durch die Umsetzung mit einem Metallsalz. Vorzugsweise erfolgt diese Umsetzung in einem Lösungsmittel, aus dem ein aus dem Metall des Metallsalzes und dem Sulfatanion gebildetes Metallsulfat auskristallisiert. Für diese Variante des Anionenaustauschs können auch die zuvor beschriebenen Hydrogensulfate eingesetzt werden. Bei dem Kation des Metallsalzes handelt es sich vorzugsweise um Alkalimetall-, Erdalkalimetall-, Blei- oder Silberionen. Das Anion des Metallsalzes ist ausgewählt unter den zuvor genannten Anionen Xⁿ⁻, wobei es sich insbesondere um ein von Cl⁻, Br⁻, I⁻ und Monoalkylsulfat verschiedenes Anion handelt. In einer geeigneten Vorgehensweise wird eine Lösung des Metallsalzes mit einer Lösung der ionischen Verbindung in Kontakt gebracht. Geeignete Lösungsmittel sind z. B. Wasser, wassermischbare Lösungsmittel, beispielsweise Alkohole, wie Methanol und Ethanol, und Mischungen davon. Die Reaktionstemperatur liegt vorzugsweise in einem Bereich von -10 bis 100 °C, insbesondere 0 bis 80 °C.

In einer weiteren Ausführungsform erfolgt der Anionenaustausch in Schritt b) durch Ionenaustauschchromatographie. Dazu eignen sich prinzipiell die dem Fachmann bekannten basischen Ionenaustauscher, die wenigstens eine an eine Festphase immobilisierte Base aufweisen. Die Festphase dieser basischen Ionenaustauscher umfasst beispielweise eine Polymermatrix. Dazu zählen z. B. Polystyrolmatrices, die neben Styrol wenigstens ein vernetzendes Monomer, z. B. Divinylbenzol, sowie gegebenenfalls weitere Comonomere einpolymerisiert enthalten. Geeignet sind weiterhin Polyacrylmatrices, die durch Polymerisation wenigstens eines (Meth)acrylats, wenigstens eines vernetzenden Monomers sowie gegebenenfalls weiterer Comonomere erhalten werden. Geeignete Polymermatrices sind auch Phenol-Formaldehyd-Harze und Polyalkylamin-Harze, die beispielsweise durch Kondensation von Polyaminen mit Epichlorhydrin erhalten werden.

Die an die Festphase direkt oder über eine Spacergruppe gebundenen so genannten Ankergruppen (deren locker gebundene Gegenionen gegen gleichsinnig geladene Ionen ausgetauscht werden können) sind vorzugsweise ausgewählt unter stickstoffhattigen Gruppen, vorzugsweise tertiären und quartären Aminogruppen.

Geeignete funktionelle Gruppen sind z. B. (geordnet nach abnehmender Basizität):

| | |
|---|---|
| -CH₂N⁺(CH₃)₃ OH⁻ | z. B. Duolite A 101 |
| -CH₂N⁺(CH₃)₂CH₂CH₂OH OH⁻ | z. B. Duolite A 102 |
| -CH₂N(CH₃)₂ | z. B. Amberlite IRA 67 |
| -CH₂NHCH₃ | |
| -CH₂NH₂ | z. B. Duolite A 365 |

Für das erfindungsgemäße Verfahren eignen sich sowohl stark als auch schwach basische Ionenaustauscher. Unter den schwach basischen Ionenaustauschern sind solche, die tertiäre Aminogruppen aufweisen bevorzugt. Stark basische Ionenaustauscher weisen in der Regel quaternäre Ammoniumgruppen als Ankergruppen auf. Für das erfindungsgemäße Verfahren geeignete kommerziell erhältliche Ionenaustauscher sind z. B. Amberlyst® A21 (Dimethylamino-funktionalisiert, schwach basisch) und Amberlyst® A27 (quaternäre Ammoniumgruppen, stark basisch). Zum Ionenaustausch werden die Ionenaustauscher zunächst mit den gewünschten Anionen Xⁿ⁻ beladen und anschliessend mit den ionische Verbindungen auf Basis von Sulfatanionen (bzw. Hydrogensulfatanionen) in Kontakt gebracht.

Das erfindungsgemäße Verfahren ermöglicht erstmals die Herstellung von Verbindungen der allgemeinen Formel b B^{m+} x Xⁿ (I), wie zuvor definiert, die frei von Cl⁻, Br⁻, I⁻ und gleichzeitig frei von Monoalkylsulfatanionen sind. Vorzugsweise erfolgt zur Herstellung von Verbindungen der Formel I mit äußerst geringem Restgehalt an Halogenidionen die Umsetzung in den Schritten a) und b) unter Ausschluss von Halogenidionen und von Materialien, die diese freisetzen. So können zur Umsetzung Reagenzien, Lösungsmittel, Inertgase etc. eingesetzt werden, die im Wesentlichen frei von Halogenidionen sind. Derartige Komponenten sind kommerziell erhältlich oder können durch übliche, dem Fachmann bekannte Reinigungsverfahren hergestellt werden. Dazu zählen z. B. Adsorptions-, Filtrations- und Ionenaustauschverfahren. Gewünschtenfalls können auch die in den Schritten a) und b) eingesetzten Vorrichtungen vor ihrem Einsatz von Halogenidionen befreit werden, z. B. durch Spülen mit halogenidfreien Lösungsmitteln. Nach dem erfindungsgemäßen Verfahren können Verbindungen der allgemeinen Formel I erhalten werden, worin Xⁿ⁻ für SO₄²⁻ steht und die einen Gesamtgehalt an Halogenidionen von höchstens 100 ppm, bevorzugt von höchstens 10 ppm und insbesondere von höchstens 1 ppm aufweisen. Des Weiteren können solche Verbindungen erhalten werden, die einen Gesamtgehalt an Monoalkylsulfatanionen von höchstens 100 ppm, bevorzugt von höchstens 10 ppm und insbesondere von höchstens 1 ppm aufweisen.

Die zuvor genannten halogenidfreien und monoalkylsulfatfreien Salze eignen sich für die Verwendung als Zwischenprodukte zur Herstellung ionischer Flüssigkeiten sowie als und zur Herstellung von Komponenten für pharmazeutische Zubereitungen. Dazu zählen beispielsweise die Salze des Clonidins :

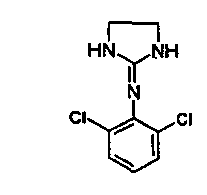

Die Erfindung wird anhand der folgenden nicht einschränkenden Beispiele näher erläutert.

### Beispiele

### Beispiel 1:

### Herstellung von 1-Butyl-3-methyl-imidazoliumsulfat durch Umsetzung in Wasser

In einem 250 ml Kolben mit Tropftrichter und Magnetrührer wurden 100 ml destilliertes Wasser und 12,6 g (0,1 mol) Dimethylsulfat vorgelegt und unter Rühren 27,3 g (0,22 mol) Butylimidazol zugetropft, wobei die Innentemperatur durch Eiskühlung auf 23 bis 25 °C gehalten wurde. Die Reaktionsmischung wurde anschließend in einen 300 ml Rührautoklaven überführt und dieser unter Rühren 6 h auf 180 °C erhitzt. Dabei stieg der Innendruck auf 8,1 bar an. Nach dem Abkühlen und Entspannen wurde der Rohaustrag am Rotationsverdampfer eingedampft und der erhaltene Rückstand am Ölpumpenvakuum bei 60 °C getrocknet, wobei eine ölige Substanz erhalten wurde. Diese wurde mit 450 ml Aceton bei Raumtemperatur 3 h gerührt und anschließend über Nacht bei Raumtemperatur stehen gelassen. Anschließend wurde unter Stickstoffatmosphäre das Aceton abgesaugt, der resultierende Feststoff nochmals mit 100 ml Aceton gewaschen und anschließend getrocknet. Es wurden 34,05 g (92,3 % der Theorie) Produkt erhalten.

### Beispiel 2:

### Herstellung von 1-Butyl-3-methyl-imidazoliumsulfat in Methanol

In einem 250 ml Kolben mit Tropftrichter und Magnetrührer wurde ein Gemisch aus 95 g Methanol und 5 g Wasser und 12,6 g (0,1 mol) Dimethylsulfat vorgelegt und unter Rühren 27,3 g (0,22 mol) Butylimidazol zugetropft, wobei die Innentemperatur durch Eiskühlung auf 23 bis 25 °C gehalten wurde. Die Reaktionsmischung wurde anschließend in einen 300 ml Rührautoklaven überführt und dieser unter Rühren 6 h auf 180 °C erhitzt. Dabei stieg der Innendruck auf 8,1 bar an. Nach dem Abkühlen und Entspannen wurde der Rohaustrag am Rotationsverdampfer eingedampft und der erhaltene Rückstand am Ölpumpenvakuum bei 60 °C getrocknet, wobei eine ölige Substanz erhalten wurde. Diese wurde mit 450 ml Aceton bei Raumtemperatur 3 h gerührt und anschließend über Nacht bei Raumtemperatur stehen gelassen. Anschließend wurde unter Stickstoffatmosphäre das Aceton abgesaugt, der resultierende Feststoff nochmals mit 100 ml Aceton gewaschen und anschließend getrocknet. Es wurden 33,7 g (90 % der Theorie) Produkt erhalten.

### Beispiel 3:

### Herstellung von 1-Methyl-3-ethyl-imidazoliumsulfat in Wasser

In einem 250 ml Rührkolben mit Tropftrichter und Magnetrührer wurden 15,4 g (0,1 mol) Diethylsulfat vorgelegt und 18,04 g (0,22 mol) Methylimidazol über 15 min. zugetropft, wobei die Innentemperatur durch Eiskühlung bei 23 bis 25 °C gehalten wurde. Anschließend wurden 100 ml destilliertes Wasser ebenfalls unter Eiskühlung innerhalb von 10 min. zugetropft und der Reaktionsansatz bei 25 °C 3 Stunden nachgerührt. Das Reaktionsgemisch wurde dann in einen 300 ml Rührautoklaven überführt und dieser unter Rühren 6 h auf 180 °C erhitzt, wobei der Innendruck auf 9,2 bar anstieg. Nach dem Abkühlen und Entspannen wird der Rohaustrag am Rotationsverdampfer eingedampft und der erhaltene Rückstand am Ölpumpenvakuum bei 60 °C getrocknet. Dabei wird eine ölige Substanz erhalten, die mit 450 ml Aceton bei Raumtemperatur 3 h gerührt und anschließend über Nacht bei Raumtemperatur stehen gelassen wird. Anschließend wird das Aceton unter Stickstoffatmosphäre abgesaugt und das resultierende Produkt gewaschen und getrocknet. Es wurden 30,65 g Produkt erhalten.

### Beispiel 4:

### Herstellung von 1-Butyl-3-methyl-imidazoliumacetat

In einem 1 I Rührkolben, der mit einem Tropftrichter ausgestattet war, wurden 31,5 g (0,1 mol) Bariumhydroxid (Octahydrat), 12 g (0,2 mol) Essigsäure und 174 g Wasser vorgelegt und auf 40 °C erwärmt. Über den Tropftrichter wurde innerhalb von 30 min. eine Lösung von 37,7 g (0,1 mol) des in Beispiel 1 erhaltenen 1-Butyl-3-methyl-imidazoliumsulfats in 339,3 g Wasser zugetropft. Sofort nach Beginn der Zugabe bildete sich ein schneeweißer feinpulvriger Niederschlag von Bariumsulfat. Nach Beendigung der Zugabe wurde das Reaktionsgemisch noch 1,5 h bei 40 °C nachgerührt, abgekühlt und der Niederschlag über eine D4-Filtemutsche abgesaugt. Die resultierende Lösung wurde am Rotationsverdampfer eingedampft und der erhaltene Rückstand bei 60 °C im Ölpumpenvakuum getrocknet. Man erhielt 35,2 g (81 % der Theorie) Produkt.

### Beispiel 5:

### Herstellung von 1-Butyl-3-methyl-imidazoliumtetraphenylborat

In einem 100 ml Rührkolben, der mit einem Tropftrichter ausgestattet war, wurde unter Stickstoffatmosphäre eine Lösung von 9,35 g (0,025 mol) des in Beispiel 1 erhaltenen 1-Butyl-3-methyl-imidazoliumsulfats in 15,9 g Methanol vorgelegt. Über den Tropftrichter wurde langsam eine Lösung von 17 g Natriumtetraphenylborat in 27,5 g Methanol zugetropft. Sofort nach Beginn der Zugabe bildete sich ein Niederschlag von Natriumsulfat. Nach Beendigung der Zugabe wurde das Reaktionsgemisch noch 2 h bei 30 °C nachgerührt, abgekühlt und der Niederschlag abfiltriert. Der Niederschlag wird mit 20 ml Methanol nachgewaschen. Die vereinigten Lösungen werden eingeengt, wobei ein weißer Feststoff ausfällt, der isoliert und getrocknet wird. Man erhielt 21,2 g (92 % der Theorie) 1-Butyl-3-methyl-imidazoliumtetraphenylborat.

### Beispiel 6:

### Herstellung von 1-Methyl-3-ethyl-imidazoliumtrimethylsilanolat

In einem 250 ml Rührkolben, der mit einem Tropftrichter ausgestattet war, wurde unter Stickstoffatmosphäre eine Lösung von 40 g (0,125 mol) des in Beispiel 3 erhaltenen 1-Methyl-3-ethyl-imidazoliumsulfats in 40 g Methanol vorgelegt. Über den Tropftrichter wurde langsam eine Lösung von 28,12 g (0,25 mol) Natriumtrimethylsilanolat in 60,2 g Methanol zugetropft. Sofort nach Beginn der Zugabe bildete sich ein Niederschlag von Natriumsulfat. Nach Beendigung der Zugabe wurde das Reaktionsgemisch noch 2 h bei 30 °C nachgerührt, abgekühlt und der Niederschlag abfiltriert. Der Niederschlag wird mit 20 ml Methanol nachgewaschen. Die vereinigten Lösungen werden eingedampft, wobei ein öliges Produkt resultiert, das getrocknet wird. Man erhielt 29,8 g (58,7 % der Theorie) 1-Methyl-3-ethyl-imidazoliumtrimethylsilanolat.

### Beispiel 7:

### Herstellung von 1-Butyl-3-methyl-imidazoliumacetat

In einem 500 ml Rührkolben, der mit einem Tropftrichter ausgestattet war, wurde unter Stickstoffatmosphäre eine Lösung von 60,18 g (0,16 mol) des in Beispiel 1 erhaltenen 1-Butyl-3-methyl-imidazoliumsulfats in 56 g Methanol vorgelegt. Über den Tropftrichter wurde innerhalb von einer Stunde eine Lösung von 26,24 g Natriumacetat in 183 g Methanol zugetropft. Sofort nach Beginn der Zugabe bildete sich ein Niederschlag von Natriumsulfat. Nach Beendigung der Zugabe wurde das Reaktionsgemisch noch 2 h bei 30 °C nachgerührt, abgekühlt und der Niederschlag abfiltriert. Der Niederschlag wird mit 20 ml Methanol nachgewaschen. Die vereinigten Lösungen werden eingeengt, wobei eine hellgelbe Flüssigkeit zurückbleibt, die im Vakuum getrocknet wird. Man erhielt 53;9 g (85 % der Theorie) 1-Butyl-3-methyl-imidazoliumacetat.

### Beispiel 8:

### Herstellung von 1-Methyl-3-ethyl-imidazoliumacetat

149,8 g (0,72 mol) 1-Methyl-3-ethyl-imidazoliumhydrogensulfat werden in 600 ml Wasser gelöst und anschließend 226,8 g (0,72 mol) Ba(OH)₂ (Octahydrat) innerhalb von 30 Minuten portionsweise zugegeben. Die Temperatur wird auf 60 °C erhöht und das Reaktionsgemisch 2 Stunden bei dieser Temperatur gerührt. Man lässt über Nacht abkühlen und filtriert das ausgefallene BaSO₄ über Celite als Filtrierhilfsmittel ab. Nach Zugabe von 43,5 g (0,72 mol) Eisessig wird das Wasser am Rotationsverdampfer entfernt und das zurückbleibende Öl mit Essigsäureethylester extrahiert. Zur Entfernung von Wasserresten wird das Öl mit n-Butanol versetzt und dieses anschließend im Vakuum abdestilliert. Es werden 108,3 g (0,636 mol) 1-Methyl-3-ethyl-imidazoliumacetat erhalten (Ausbeute: 88 % bezogen auf 1-Methyl-3-ethyl-imidazoliumhydrogensulfat). Der Chloridgehalt beträgt 4 ppm.

### Beispiel 9:

### Herstellung von 1-Methyl-3-ethyl-imidazoliumacetat

226,8 g (0,72 mol) Ba(OH)₂ (Octahydrat) werden in 600 g Wasser suspendiert. Man heizt auf 80 °C auf, wobei das Bariumsalz schmilzt und als wässrige Emulsion vorliegt. In diese Emulsion tropft man 149,8 g (0,72 mol) 1-Methyl-3-ethyl-imidazolium-hydrogensulfat, wobei die Temperatur auf 100 °C ansteigt. Trotz des dabei ausfallenden Bariumsulfats bleibt die Suspension gut rührbar. Das Reaktionsgemisch wird noch 2 Stunden bei 80 °C gerührt, abgekühlt und das ausgefallene BaSO₄ über Celite als Filtrierhilfsmittel abfiltriert. Nach Zugabe von 43,5 g (0,72 mol) Eisessig wird das Wasser am Rotationsverdampfer entfernt und das zurückbleibende Öl mit Essigsäureethylester extrahiert. Nach Trocknen im Vakuum werden 113,3 g (0,67 mol) 1-Methyl-3-ethyl-imidazoliumacetat erhalten (Ausbeute: 92 %). Der Chloridgehalt beträgt 4 ppm.

### Beispiel 10:

### Herstellung von 1-Methyl-3-ethyl-imidazoliumdihydrogenphosphat

208 g (1,0 mol) 1-Methyl-3-ethyl-imidazoliumhydrogensulfat werden in 600 ml Wasser gelöst und anschließend 315,3 g (1,0 mol) Ba(OH)₂ (Octahydrat) innerhalb von 30 Minuten portionsweise zugegeben. Die Temperatur wird auf 60 °C erhöht und das Reaktionsgemisch 2 Stunden bei dieser Temperatur gerührt. Man lässt über Nacht abkühlen und filtriert das ausgefallene BaSO₄ über Celite als Filtrierhilfsmittel ab. Nach Zugabe von 115,3 g (1,0 mol) 85%iger Phosphorsäure wird das Wasser am Rotationsverdampfer entfernt. Man erhält einen weißen Feststoff (202,8 g, 0,975 mol) mit einem Schmelzpunkt von 140 °C (Ausbeute: 98 % bezogen auf 1-Methyl-3-ethyl-imidazolium-hydrogensulfat). Der Chloridgehalt beträgt 4 ppm.

### Beispiel 11:

### Herstellung von 1-Methyl-3-ethyl-imidazoliumdihydrogenborat

631 g (2,0 mol) Ba(OH)₂ (Octahydrat) und 123,6 g (2,0 mol) Borsäure werden bei 60 °C in 500 ml Wasser suspendiert. In diese Mischung tropft man innerhalb von 60 Minuten 208 g (1,0 mol) 1-Methyl-3-ethyl-imidazoliumhydrogensulfat. Nach Zugabe von weiteren 500 ml Wasser wird das ausgefallene BaSO₄ über Celite als Filtrierhilfsmittel abfiltriert und mit Wasser nachgewaschen. Nach Zugabe von n-Butanol wird das Wasser am Rotationsverdampfer entfernt und nach Trocknen im Vakuum werden 247,2 g (1,44 mol) 1-Methyl-3-ethyl-imidazoliumdihydrogenborat erhalten (Ausbeute: 72 %, Schmelzpunkt: 40 °C).

## Patentansprüche

1. Verfahren zur Herstellung einer ionischen Verbindung, die wenigstens ein Kation mit einem quaternären sp²-hybridisierten Stickstoffatom umfasst, bei dem man
a) eine Verbindung, die ein doppelt gebundenes Stickstoffatom enthält, mit einem Dialkylsulfat unter Einsatz beider Alkylgruppen des Dialkylsulfats umsetzt, wobei eine ionische Verbindung mit Sulfatanionen erhalten wird, und
b) die in Schritt a) erhaltene ionische Verbindung gegebenenfalls einem Anionenaustausch unterzieht.

2. Verfahren nach Anspruch 1, wobei sich das Kation von Iminen, Diazenen, Amidinen, Amidoximen, Amidrazonen, Oximen, Sulfimiden, Guanidinen, Phosphiniminen oder Stickstoff-haltigen aromatischen Heterocyclen ableitet.

3. Verfahren nach einem der Ansprüche 1 oder 2, wobei die erhaltene ionische Verbindung wenigstens ein Anion Xⁿ⁻ umfasst, worin n für eine ganze Zahl entsprechend der Wertigkeit des Anions steht und das ausgewählt ist unter SO₄², HSO₄⁻, NO₂⁻, NO₃⁻, CN⁻, OCN⁻, NCO⁻, SCN⁻, NCS⁻, PO₄³-, HPO₄²⁻, (H₂PO₄⁻), H₂PO₃⁻, HPO₃²⁻, BO₃³⁻, (BO₂)₃³⁻, [BF₄]⁻, [BCl₄]⁻, [B(C₆H₅)₄]⁻, [PF₆]⁻, [SbF₆]⁻, [AsF₆]⁻, [AlCl₄]⁻, [AlBr₄]⁻, [ZnCl₃]⁻, Dichlorocupraten(I) und (II), CO₃²⁻, HCO₃⁻_{,} F⁻, (R'-COO)⁻, R'₃SiO⁻, (R'-SO₃)⁻ und [(R'-SO₂)₂N]⁻, worin R' für Alkyl, Cycloalkyl oder Aryl steht.

4. Verfahren nach einem der vorhergehenden Ansprüche zur Herstellung von Verbindungen der allgemeinen Formel II 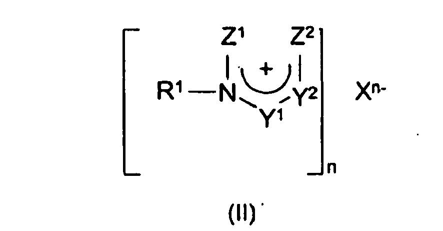 worin
R¹ für C₁- bis C₁₀-Alkyl steht,
Y¹ und Y² unabhängig voneinander ausgewählt sind unter Heteroatomen und heteroatomhaltigen Gruppe, die jeweils ein freies Elektronenpaar aufweisen, sowie Gruppen CR², worin das Kohlenstoffatom sp²-hybridisiert ist und R² für Wasserstoff oder einen Organylrest steht,
Z¹ und Z² unabhängig voneinander für einfach oder doppelt gebundene Organylreste stehen, wobei Z¹ und Z² auch gemeinsam für eine verbrückende Gruppe mit 2 bis 5 Atomen zwischen den flankierenden Bindungen stehen können,
Xⁿ⁻ für ein Anion steht, das vorzugsweise von Cl⁻, Br⁻, I⁻ und Monoalkylsulfat verschieden ist, und
n für eine ganze Zahl von 1 bis 3 steht,
wobei die Gruppe NR¹-Y¹-Y² und gegebenenfalls zusätzlich Z¹ und/oder Z² Teil eines delokalisierten π-Elektronensystems sind,
bei dem man
a) eine Verbindung der allgemeinen Formel 11.1 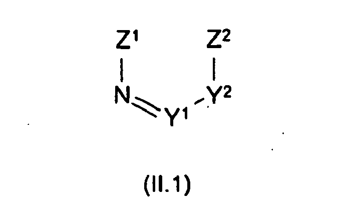 worin Y¹, Y², Z¹ und Z² die angegebenen Bedeutungen besitzen, mit einem Dialkylsulfat (R¹)₂SO₄, worin R¹ für C₁- bis C₁₀-Alkyl steht, bei erhöhter Temperatur und unter Einsatz beider Alkylgruppen des Dialkylsulfats zu einer Verbindung der Formel II umsetzt, worin Xⁿ⁻ für ein Sulfatanion steht, und
b) gegebenenfalls das Sulfatanion gegen ein davon verschiedenes Anion austauscht.

5. Verfahren nach Anspruch 4, wobei in den Formeln II und 11.1 die Gruppen Y¹ und Y² unabhängig voneinander ausgewählt sind unter O, S, CR², NR³ oder PR⁴, worin R², R³ und R⁴ unabhängig voneinander für Wasserstoff, Alkyl, substituierte Alkylgruppen, welche 1, 2, 3, 4 oder 5 Substituenten aufweisen, die ausgewählt sind unter Cycloalkyl, Aryl, Hetaryl, Halogen, Amino, Alkoxycarbonyl, Acyl, Nitro, Aminocarbonyl, Alkylaminocarbonyl, Dialkylaminocarbonyl, Alkylcarbonylamino, Carboxylat und Sulfonat, Cycloalkyl, substituierte Cycloalkylgruppen, die 1, 2, 3, 4 oder 5 Substituenten tragen, die ausgewählt sind unter Alkyl und den zuvor für substituierte Alkylgruppen genannten Substituenten, Heterocycloalkyl, substituierte heterocycloaliphatische Gruppen, die 1, 2 oder 3 Substituenten tragen, die ausgewählt sind unter Alkyl und den zuvor für substituierte Alkylgruppen genannten Substituenten, Aryl, substituierte Arylgruppen, die 1, 2, 3, 4 oder 5 Substituenten tragen, die ausgewählt sind unter Alkyl und den zuvor für substituierte Alkylgruppen genannten Substituenten, Hetaryl, substituierte heterocycloaromatische Gruppen, die 1, 2 oder 3 Substituenten tragen, die ausgewählt sind unter Alkyl und den zuvor für substituierte Alkylgruppen genannten Substituenten, COOR^{a}, COO⁻M⁺, SO₃R^{a}, SO₃⁻M⁺, Sulfonamid, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, Alkylaminocarbonyl, Dialkylaminocarbonyl, Alkylcarbonylamino, Halogen, Nitro, Acyl oder Cyano stehen, wobei
R^{a} jeweils gleiche oder verschiedene Reste, ausgewählt unter Wasserstoff, Alkyl, substituierten Alkylgruppen, welche 1, 2, 3, 4 oder 5 Substituenten aufweisen, die ausgewählt sind unter Cycloalkyl, Aryl, Hetaryl, Halogen, Amino, Alkoxycarbonyl, Acyl, Nitro, Aminocarbonyl, Alkylaminocarbonyl, Dialkylaminocarbonyl, Alkylcarbonylamino, Carboxylat und Sulfonat, Cycloalkyl, substituierten Cycloalkylgruppen, die 1, 2, 3, 4 oder 5 Substituenten tragen, die ausgewählt sind unter Alkyl und den zuvor für substituierte Alkylgruppen genannten Substituenten, Aryl, substituierten Arylgruppen, die 1, 2, 3, 4 oder 5 Substituenten tragen, die ausgewählt sind unter Alkyl und den zuvor für substituierte Alkylgruppen genannten Substituenten, Hetaryl oder substituierten heterocycloaromatischen Gruppen, die 1, 2 oder 3 Substituenten tragen, die ausgewählt sind unter Alkyl und den zuvor für substituierte Alkylgruppen genannten Substituenten, bedeuten,
E¹, E², E³ jeweils gleiche oder verschiedene Reste, ausgewählt unter Wasserstoff, Alkyl, substituierten Alkylgruppen, welche 1, 2, 3, 4 oder 5 Substituenten aufweisen, die ausgewählt sind unter Cycloalkyl, Aryl, Hetaryl, Halogen, Amino, Alkoxycarbonyl, Acyl, Nitro, Aminocarbonyl, Alkylaminorarbonyl, Dialkylaminocarbonyl, Alkylcarbonylamino, Carboxylat und Sulfonat, Cycloalkyl, substituierten Cycloalkylgruppen, die 1, 2, 3, 4 oder 5 Substituenten tragen, die ausgewählt sind unter Alkyl und den zuvor für substituierte Alkylgruppen genannten Substituenten, Aryl, substituierten Arylgruppen, die 1, 2, 3, 4 oder 5 Substituenten tragen, die ausgewählt sind unter Alkyl und den zuvor für substituierte Alkylgruppen genannten Substituenten, Hetaryl oder substituierten heterocycloaromatischen Gruppen, die 1, 2 oder 3 Substituenten tragen, die ausgewählt sind unter Alkyl und den zuvor für substituierte Alkylgruppen genannten Substituenten, bedeuten,
R^{b} für Wasserstoff, Methyl oder Ethyl steht,
M⁺ für ein Kationäquivatent steht,
A⁻ für ein Anionäquivalent steht und
y für eine ganze Zahl von 1 bis 250 steht.

6. Verfahren nach einem der Ansprüche 4 oder 5, wobei Z¹ und Z² gemeinsam für eine verbrückende Gruppe stehen mit zwei bis drei Atomen zwischen den flankierenden Bindungen, die ausgewählt sind unter gegebenenfalls substituierten Heteroatomen und sp²-hybridisierten Kohlenstoffatomen, wobei die verbrückende Gruppe gemeinsam mit der Gruppe NR¹-Y¹-Y² ein delokalisiertes π-Elektronensystem bildet.

7. Verfahren nach einem der Ansprüche 4 bis 6, wobei die Verbindung der Formel II ausgewählt ist unter Verbindungen der Formel II.a bis II.e 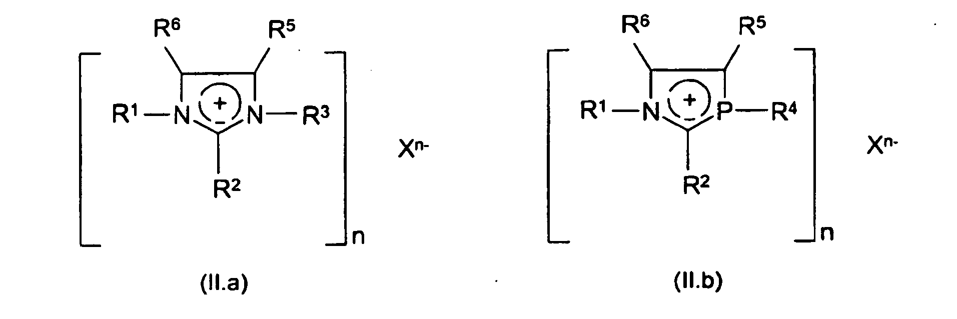 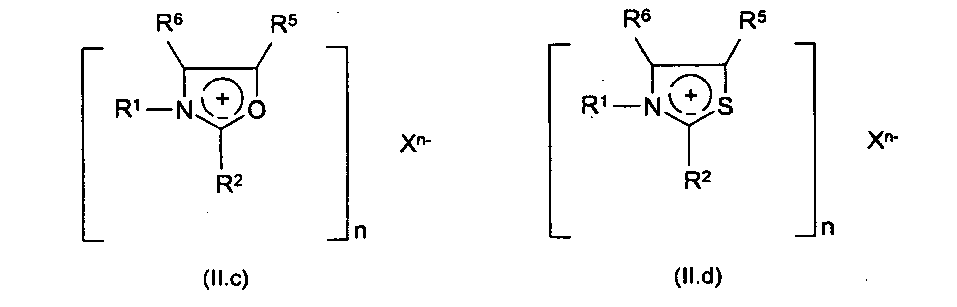 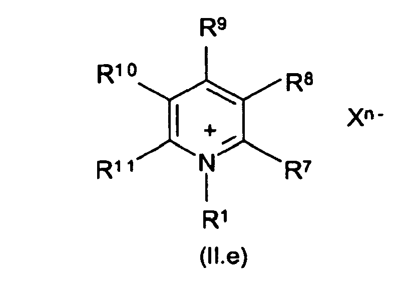 worin
Xⁿ⁻ für ein Anion steht, das vorzugsweise von Cl⁻, Br⁻, I⁻ und Monoalkylsulfat verschieden ist, und,
n für eine ganze Zahl von 1 bis 3 steht,
R¹ für C₁- bis C₁₀-Alkyl steht, und
R², R³ ,R⁴, R⁵, R⁶ ,R⁷, R⁸, R⁹ ,R¹⁰ und R¹¹ unabhängig voneinander für Wasserstoff, Alkyl, substituierte Alkylgruppen, welche 1, 2, 3, 4 oder 5 Substituenten aufweisen, die ausgewählt sind unter Cycloalkyl, Aryl, Hetaryl, Halogen, Amino, Alkoxycarbonyl, Acyl, Nitro, Aminocarbonyl, Alkylaminocarbonyl, Dialkylaminocarbonyl, Alkylcarbonylamino, Carboxylat und Sulfonat, Cycloalkyl, substituierte Cycloalkylgruppen, die 1, 2, 3, 4 oder 5 Substituenten tragen, die ausgewählt sind unter Alkyl und den zuvor für substituierte Alkylgruppen genannten Substituenten, Heterocycloalkyl, substituierte heterocycloaliphatische Gruppen, die 1, 2 oder 3 Substituenten tragen, die ausgewählt sind unter Alkyl und den zuvor für substituierte Alkylgruppen genannten Substituenten, Aryl, substituierte Arylgruppen, die 1, 2, 3, 4 oder 5 Substituenten tragen, die ausgewählt sind unter Alkyl und den zuvor für substituierte Alkylgruppen genannten Substituenten, Hetaryl, substituierte heterocycloaromatische Gruppen, die 1, 2 oder 3 Substituenten tragen, die ausgewählt sind unter Alkyl und den zuvor für substituierte Alkylgruppen genannten Substituenten, COOR^{a}, COO⁻M⁺, SO₃R^{a}, SO₃⁻M⁺, Sulfonamid, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, Alkylaminocarbonyl, Dialkylaminocarbonyl, Alkylcarbonylamino, Halogen, Nitro, Acyl oder Cyano stehen, wobei
R^{a} jeweils gleiche oder verschiedene Reste, ausgewählt unter Wasserstoff, Alkyl, substituierten Alkylgruppen, welche 1, 2, 3, 4 oder 5 Substituenten aufweisen, die ausgewählt sind unter Cycloalkyl, Aryl, Hetaryl, Halogen, Amino, Alkoxycarbonyl, Acyl, Nitro, Aminocarbonyl, Alkylaminocarbonyl, Dialkylaminocarbonyl, Alkylcarbonylamino, Carboxylat und Sulfonat, Cycloalkyl, substituierten Cycloalkylgruppen, die 1, 2, 3, 4 oder 5 Substituenten tragen, die ausgewählt sind unter Alkyl und den zuvor für substituierte Alkylgruppen genannten Substituenten, Aryl, substituierten Arylgruppen, die 1, 2, 3, 4 oder 5 Substituenten tragen, die ausgewählt sind unter Alkyl und den zuvor für substituierte Alkylgruppen genannten Substituenten, Hetaryl oder substituierten heterocycloaromatischen Gruppen, die 1, 2 oder 3 Substituenten tragen, die ausgewählt sind unter Alkyl und den zuvor für substituierte Alkylgruppen genannten Substituenten, bedeuten,
E¹, E², E³ jeweils gleiche oder verschiedene Reste, ausgewählt unter Wasserstoff, Alkyl, substituierten Alkylgruppen, welche 1, 2, 3, 4 oder 5 Substituenten aufweisen, die ausgewählt sind unter Cycloalkyl, Aryl, Hetaryl, Halogen, Amino, Alkoxycarbonyl, Acyl, Nitro, Aminocarbonyl, Alkylaminocarbonyl, Dialkylaminocarbonyl, Alkylcarbonylamino, Carboxylat und Sulfonat, Cycloalkyl, substituierten Cycloalkylgruppen, die 1, 2, 3, 4 oder 5 Substituenten tragen, die ausgewählt sind unter Alkyl und den zuvor für substituierte Alkylgruppen genannten Substituenten, Aryl, substituierten Arylgruppen, die 1, 2, 3, 4 oder 5 Substituenten tragen, die ausgewählt sind unter Alkyl und den zuvor für substituierte Alkylgruppen genannten Substituenten, Hetaryl oder substituierten heterocycloaromatischen Gruppen, die 1, 2 oder 3 Substituenten tragen, die ausgewählt sind unter Alkyl und den zuvor für substituierte Alkylgruppen genannten Substituenten, bedeuten,
R^{b} für Wasserstoff, Methyl oder Ethyl steht,
M⁺ für ein Kationäquivalent steht,
A⁻ für ein Anionäquivalent steht und
y für eine ganze Zahl von 1 bis 250 steht.

8. Verfahren nach einem der vorhergehenden Ansprüche, wobei die Umsetzung in Schritt a) bei einer Temperatur wenigstens 60 °C, bevorzugt wenigstens 80 °C, insbesondere im Bereich von 100 bis 220 °C erfolgt.

9. Verfahren nach einem der vorhergehenden Ansprüche, wobei das Molmengenverhältnis von der Verbindung, die ein doppelt gebundenes Stickstoffatom enthält, zu dem Dialkylsulfat wenigstens 2 : 1 beträgt.

10. Verfahren nach einem der vorhergehenden Ansprüche, wobei die Umsetzung in Schritt a) in einem organischen Lösungsmittel, in Wasser oder in einer Mischung davon durchgeführt wird.

11. Verfahren nach Anspruch 10, wobei das Lösungsmittel mindestens 30 Vol.-% Wasser umfasst.

12. Verfahren nach einem der vorhergehenden Ansprüche, wobei die Umsetzung in Schritt a) in Gegenwart eines Inertgases erfolgt.

13. Verfahren nach einem der vorhergehenden Ansprüche, bei dem das Dialkylsulfat unter Dimethylsulfat und Diethylsulfat ausgewählt ist.

14. Verfahren nach einem der vorhergehenden Ansprüche, wobei die Verfahrensschritte a) und b) in Abwesenheit von Halogenidionen durchgeführt werden.

15. Verfahren nach einem der vorhergehenden Ansprüche, wobei der Austausch des Sulfatanions in Schritt b) durch Umprotonierung mit H₂SO₄, Umsetzung mit einem Metallsalz, Ionenaustauschchromatographie oder eine Kombination davon erfolgt.

16. Verfahren nach Anspruch 15, wobei die Umsetzung mit dem Metallsalz in einem Lösungsmittel durchgeführt wird, aus dem ein aus dem Metall des Metallsalzes und dem Sulfatanion gebildetes Metallsulfat auskristallisiert.

## Claims

1. A process for preparing an ionic compound comprising at least one cation containing a quaternary sp²-hybridized nitrogen atom, which comprises
a) reacting a compound containing a double-bonded nitrogen atom with a dialkyl sulfate with participation of both alkyl groups of the dialkyl sulfate to give an ionic compound containing sulfate anions, and
b) if appropriate, subjecting the ionic compound obtained in step a) to an anion exchange.

2. The process according to claim 1, wherein the cation is derived from imines, diazines, amidines, amidoximes, amidrazones, oximes, sulfimides, guanidines, phosphinimines or nitrogen-containing aromatic heterocycles.

3. The process according to claim 1 or 2, wherein the ionic compound obtained comprises at least one anion Xⁿ⁻ in which n is an integer corresponding to the valence of the anion and which is selected from among SO₄²⁻, HSO₄⁻, NO₂⁻, NO₃⁻, CN⁻, OCN⁻, NCO⁻, SCN⁻, NCS⁻, PO₄³⁻, HPO₄²⁻, H₂PO₄⁻, H₂PO₃⁻, HPO₃²⁻, BO₃³⁻, (BO₂)₃³⁻, [BF₄]⁻, [BCl₄]⁻, [B(C₆H₅)₄]⁻, [PF₆]⁻, [SbF₆]⁻, [AsF₆]⁻, [AlCl₄]⁻, [AlBr₄]⁻, [ZnCl₃]⁻, dichlorocuprates (I) and (II), CO₃²⁻, HCO₃⁻, F⁻, (R'-COO)⁻, R⁻₃SiO⁻, R'-SO₃⁻ and [(R'-SO₂)₂N]⁻, where R' is alkyl, cycloalkyl or aryl.

4. The process according to any of the preceding claims for preparing compounds of the general formula II 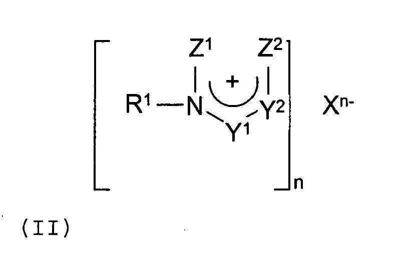 where
R¹ is C₁-C₁₀-alkyl,
Y¹ and Y² are selected independently from among heteroatoms and heteroatom-containing groups which each have a free electron pair and groups CR² in which the carbon atom is sp²-hybridized and R² is hydrogen or an organyl radical,
Z¹ and Z² are each, independently of one another, a single- or double-bonded organyl radical, where Z¹ and Z² may also together form a bridging group having from 2 to 5 atoms between the flanking bonds,
Xⁿ⁻ is an anion which is preferably not Cl⁻, Br⁻, I⁻ or monoalkylsulfate, and
n is an integer from 1 to 3,
where the group NR¹-Y¹-Y² and, if appropriate, also Z¹ and/or Z² are part of a delocalized π electron system,
wherein
a) a compound of the general formula II.1 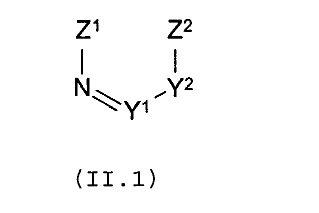 where Y¹, Y², Z¹ and Z² are as defined above, is reacted with a dialkyl sulfate (R¹)₂SO₄, where R¹ is C₁-C₁₀-alkyl, at elevated temperature with participation of both alkyl groups of the dialkyl sulfate to form a compound of the formula II in which Xⁿ⁻ is a sulfate anion, and
b) if appropriate, the sulfate anion is exchanged for a different anion.

5. The process according to claim 4, wherein the groups Y¹ and Y² in the formulae II and II.1 are selected independently from among O, S, CR², NR³ and PR⁴, where R², R³ and R⁴ are each, independently of one another, hydrogen, alkyl, a substituted alkyl group having 1, 2, 3, 4 or 5 substituents selected from among cycloalkyl, aryl, hetaryl, halogen, amino, alkoxycarbonyl, acyl, nitro, aminocarbonyl, alkylaminocarbonyl, dialkylaminocarbonyl, alkylcarbonylamino, carboxylate and sulfonate, cycloalkyl, a substituted cycloalkyl group bearing 1, 2, 3, 4 or 5 substituents selected from among alkyl and the substituents mentioned above for substituted alkyl groups, heterocycloalkyl, a substituted heterocycloaliphatic group bearing 1, 2 or 3 substituents selected from among alkyl and the substituents mentioned above for substituted alkyl groups, aryl, a substituted aryl group bearing 1, 2, 3, 4 or 5 substituents selected from among alkyl and the substituents mentioned above for substituted alkyl groups, hetaryl, a substituted heterocycloaromatic group bearing 1, 2 or 3 substituents selected from among alkyl and the substituents mentioned above for substituted alkyl groups, COOR^{a}, COO⁻M⁺, SO₃R^{a}, SO₃⁻M⁺, sulfonamide, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, alkylaminocarbonyl, dialkylaminocarbonyl, alkylcarbonylamino, halogen, nitro, acyl or cyano, where
the radicals R^{a} are identical or different and are selected from among hydrogen, alkyl, substituted alkyl groups having 1, 2, 3, 4 or 5 substituents selected from among cycloalkyl, aryl, hetaryl, halogen, amino, alkoxycarbonyl, acyl, nitro, aminocarbonyl, alkylaminocarbonyl, dialkylaminocarbonyl, alkylcarbonylamino, carboxylate and sulfonate, cycloalkyl, substituted cycloalkyl groups bearing 1, 2, 3, 4 or 5 substituents selected from among alkyl and the substituents mentioned above for substituted alkyl groups, aryl, substituted aryl groups bearing 1, 2, 3, 4 or 5 substituents selected from among alkyl and the substituents mentioned above for substituted alkyl groups, hetaryl and substituted heterocycloaromatic groups bearing 1, 2 or 3 substituents selected from among alkyl and the substituents mentioned above for substituted alkyl groups,
E¹, E², E³ are identical or different radicals selected from among hydrogen, alkyl, substituted alkyl groups having 1, 2, 3, 4 or 5 substituents selected from among cycloalkyl, aryl, hetaryl, halogen, amino, alkoxycarbonyl, acyl, nitro, aminocarbonyl, alkylaminocarbonyl, dialkylaminocarbonyl, alkylcarbonylamino, carboxylate and sulfonate, cycloalkyl, substituted cycloalkyl groups bearing 1, 2, 3, 4 or 5 substituents selected from among alkyl and the substituents mentioned above for substituted alkyl groups, aryl, substituted aryl groups bearing 1, 2, 3, 4 or 5 substituents selected from among alkyl and the substituents mentioned above for substituted alkyl groups, hetaryl and substituted heterocycloaromatic groups bearing 1, 2 or 3 substituents selected from among alkyl and the substituents mentioned above for substituted alkyl groups,
R^{b} is hydrogen, methyl or ethyl,
M⁺ is a cation equivalent,
A⁻ is an anion equivalent and
y is an integer from 1 to 250.

6. The process according to claim 4 or 5, wherein Z¹ and Z² together form a bridging group having two or three atoms between the flanking bonds, which are selected from among optionally substituted heteroatoms and sp²-hybridized carbon atoms, with the bridging group together with the group NR¹-Y¹-Y² forming a delocalized π electron system.

7. The process according to any of claims 4 to 6,
wherein the compound of the formula II is selected from among compounds of the formulae II.a to II.e 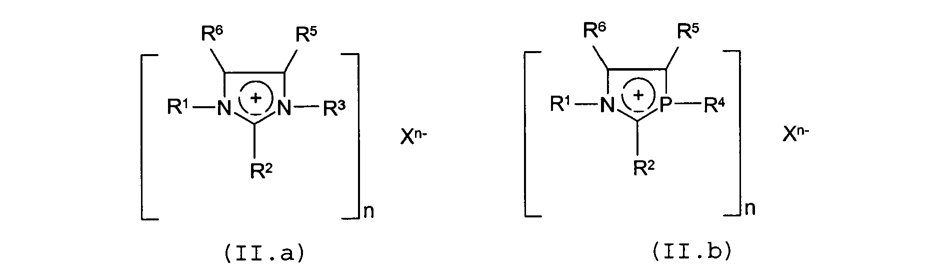 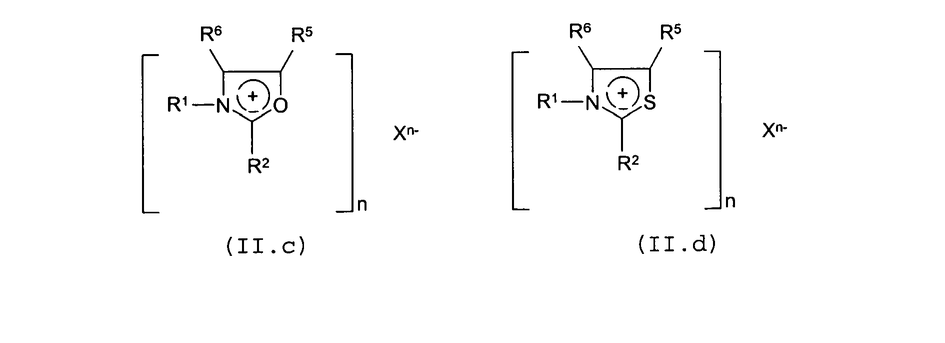 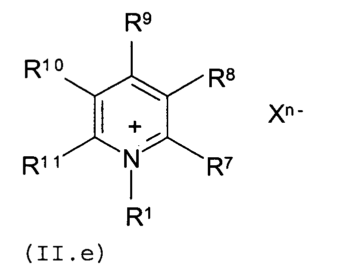 where
Xⁿ⁻ is an anion which is preferably not Cl⁻, Br⁻, I⁻ nor monoalkylsulfate, and
n is an integer from 1 to 3,
R¹ is C₁-C₁₀-alkyl, and
R², R³, R⁴, R⁵, R⁶, R⁷, R⁸, R⁹, R¹⁰ and R¹¹ are each, independently of one another, hydrogen, alkyl, a substituted alkyl group having 1, 2, 3, 4 or 5 substituents selected from among cycloalkyl, aryl, hetaryl, halogen, amino, alkoxycarbonyl, acyl, nitro, aminocarbonyl, alkylaminocarbonyl, dialkylaminocarbonyl, alkylcarbonylamino, carboxylate and sulfonate, cycloalkyl, a substituted cycloalkyl group bearing 1, 2, 3, 4 or 5 substituents selected from among alkyl and the substituents mentioned above for substituted alkyl groups, heterocycloalkyl, a substituted heterocycloaliphatic group bearing 1, 2 or 3 substituents selected from among alkyl and the substituents mentioned above for substituted alkyl groups, aryl, a substituted aryl group bearing 1, 2, 3, 4 or 5 substituents selected from among alkyl and the substituents mentioned above for substituted alkyl groups, hetaryl, a substituted heterocycloaromatic group bearing 1, 2 or 3 substituents selected from among alkyl and the substituents mentioned above for substituted alkyl groups, COOR^{a}, COO⁻M⁺, SO₃R^{a}, SO₃⁻M⁺, sulfonamide, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, alkylaminocarbonyl, dialkylaminocarbonyl, alkylcarbonylamino, halogen, nitro, acyl or cyano, where
the radicals R^{a} are identical or different and are selected from among hydrogen, alkyl, substituted alkyl groups having 1, 2, 3, 4 or 5 substituents selected from among cycloalkyl, aryl, hetaryl, halogen, amino, alkoxycarbonyl, acyl, nitro, aminocarbonyl, alkylaminocarbonyl, dialkylaminocarbonyl, alkylcarbonylamino, carboxylate and sulfonate, cycloalkyl, substituted cycloalkyl groups bearing 1, 2, 3, 4 or 5 substituents selected from among alkyl and the substituents mentioned above for substituted alkyl groups, aryl, substituted aryl groups bearing 1, 2, 3, 4 or 5 substituents selected from among alkyl and the substituents mentioned above for substituted alkyl groups, hetaryl and substituted heterocycloaromatic groups bearing 1, 2 or 3 substituents selected from among alkyl and the substituents mentioned above for substituted alkyl groups,
E¹, E², E³ are identical or different radicals selected from among hydrogen, alkyl, substituted alkyl groups having 1, 2, 3, 4 or 5 substituents selected from among cycloalkyl, aryl, hetaryl, halogen, amino, alkoxycarbonyl, acyl, nitro, aminocarbonyl, alkylaminocarbonyl, dialkylaminocarbonyl, alkylcarbonylamino, carboxylate and sulfonate, cycloalkyl, substituted cycloalkyl groups bearing 1, 2, 3, 4 or 5 substituents selected from among alkyl and the substituents mentioned above for substituted alkyl groups, aryl, substituted aryl groups bearing 1, 2, 3, 4 or 5 substituents selected from among alkyl and the substituents mentioned above for substituted alkyl groups, hetaryl and substituted heterocycloaromatic groups bearing 1, 2 or 3 substituents selected from among alkyl and the substituents mentioned above for substituted alkyl groups,
R^{b} is hydrogen, methyl or ethyl,
M⁺ is a cation equivalent,
A⁻ is an anion equivalent and
y is an integer from 1 to 250.

8. The process according to any of the preceding claims, wherein the reaction in step a) is carried out at a temperature of at least 60°C, preferably at least 80°C, in particular in the range from 100 to 220°C.

9. The process according to any of the preceding claims, wherein the molar ratio of the compound containing a double-bonded nitrogen atom to the dialkyl sulfate is at least 2:1.

10. The process according to any of the preceding claims, wherein the reaction in step a) is carried out in an organic solvent, in water or in a mixture thereof.

11. The process according to claim 10, wherein the solvent comprises at least 30% by volume of water.

12. The process according to any of the preceding claims, wherein the reaction in step a) is carried out in the presence of an inert gas.

13. The process according to any of the preceding claims, wherein the dialkyl sulfate is selected from among dimethyl sulfate and diethyl sulfate.

14. The process according to any of the preceding claims, wherein the process steps a) and b) are carried out in the absence of halide ions.

15. The process according to any of the preceding claims, wherein the exchange of the sulfate anion in step b) is effected by transprotonation with H₂SO₄, reaction with a metal salt, ion exchange chromatography or a combination thereof.

16. The process according to claim 15, wherein the reaction with the metal salt is carried out in a solvent from which a metal sulfate formed from the metal of the metal salt and the sulfate anion crystallizes out.

## Revendications

1. Procédé pour la préparation d'un composé ionique, qui comprend au moins un cation avec un atome d'azote hybridé en sp² quaternaire, dans lequel
a) on transforme un composé qui contient un atome d'azote doublement lié, avec un dialkylsulfate avec utilisation des deux groupes alkyle du dialkylsulfate, avec obtention d'un composé ionique avec des anions sulfate, et
b) on soumet le composé ionique contenu dans l'étape
a) le cas échéant à un échange anionique.

2. Procédé selon la revendication 1, le cation étant dérivé d'imines, de diazènes, d'amidines, d'amidoximes, d'amidrazones, d'oximes, de sulfimides, de guanidines, de phosphinimines ou d'hétérocycles aromatiques contenant de l'azote.

3. Procédé selon l'une quelconque des revendications 1 ou 2, où le composé ionique obtenu comprend au moins un anion Xⁿ⁻, où n représente un nombre entier correspondant à la valence de l'anion et qui est choisi parmi SO₄^{2-,} HSO₄⁻, NO₂⁻, NO₃⁻, CN⁻, OCN⁻, NCO⁻, SCN⁻, NCS⁻, PO₄³⁻, HPO₄²⁻, (H₂PO₄⁻), H₂PO₃⁻, HPO₃²⁻, BO₃³⁻, (BO₂)₃³⁻, (BF₄]⁻, [BCl₄]⁻, [B(C₆H₅)₄]⁻, [PF₆]⁻, [SbF₆]⁻, (AsF₆]⁻, [AlCl₄]⁻, [AlBr₄]⁻, [ZnCl₃]⁻, dichlorocuprates (I) et (II), CO₃²⁻, HCO₃⁻, F⁻, (R'-COO)⁻, R'₃SiO⁻, (R'-SO₃)⁻ et [(R'-SO₂)₂N]⁻, où R' représente alkyle, cycloalkyle ou aryle.

4. Procédé selon l'une quelconque des revendications précédentes pour la préparation de composés de formule générale II : 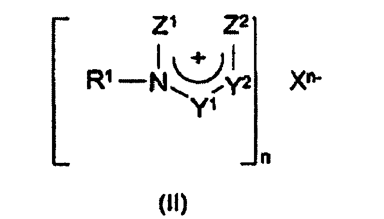 où
R¹ représente alkyle en C₁-C₁₀,
Y¹ et Y² sont choisis, indépendamment l'un de l'autre, parmi les hétéroatomes et les groupes contenant des hétéroatomes, qui présentent à chaque fois une paire d'électrons libres, ainsi que les groupes CR², où l'atome de carbone est hybridé en sp² et R² représente hydrogène ou un radical organyle,
Z¹ et Z² représentent, indépendamment l'un de l'autre, des radicaux organyle simplement ou doublement liés, où Z¹ et Z² peuvent également représenter, ensemble, un groupe formant un pont comprenant 2 à 5 atomes entre les liaisons adjacentes,
Xⁿ⁻ représente un anion qui est de préférence différent de Cl⁻, Br⁻, I⁻ et monoalkylsulfate, et
n vaut un nombre entier de 1 à 3,
où le groupe NR¹-Y¹-Y² et le cas échéant Z¹ et/ou Z² font partie d'un système d'électrons π délocalisés,
dans lequel
a) on transforme un composé de formule générale II.1 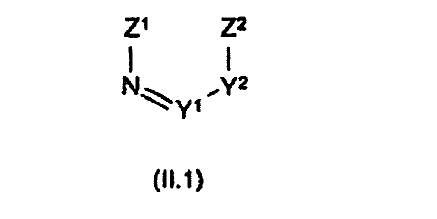 où Y¹, Y², Z¹ et Z² présentent les significations indiquées, avec un dialkylsulfate (R¹)₂SO₄, où R¹ représente alkyle en C₁ à C₁₀, à température élevée et avec utilisation des deux groupes alkyle du dialkylsulfate en un composé de formule II, où Xⁿ⁻ représente un anion sulfate, et
b) on remplace le cas échéant l'anion sulfate par un anion différent de celui-ci.

5. Procédé selon la revendication 4, où, dans les formules II et II.1, les groupes Y¹ et Y² sont choisis indépendamment l'un de l'autre, parmi O, S, CR², NR³ ou PR⁴, où R², R³ et R⁴ représentent, indépendamment l'un de l'autre, hydrogène, alkyle, des groupes alkyle substitués, qui présentent 1, 2, 3, 4 ou 5 substituants, qui sont choisis parmi des groupes cycloalkyle, aryle, hétaryle, halogène, amino, alcoxycarbonyle, acyle, nitro, aminocarbonyle, alkylaminocarbonyle, dialkylaminocarbonyle, alkylcarbonylamino, carboxylate et sulfonate, cycloalkyle, des groupes cycloalkyle substitués, qui portent 1, 2, 3, 4 ou 5 substituants, qui sont choisis parmi alkyle et les substituants mentionnés ci-dessus pour les groupes alkyle substitués, hétérocycloalkyle, des groupes hétérocycloaliphatiques substitués, qui portent 1, 2 ou 3 substituants, qui sont choisis parmi alkyle et les substituants mentionnés ci-dessus pour les groupes alkyle substitués, aryle, des groupes aryle substitués, qui portent 1, 2, 3, 4 ou 5 substituants, qui sont choisis parmi alkyle et les substituants mentionnés ci-dessus pour les groupes alkyle substitués, hétaryle, des groupes hétérocycloaromatiques substitués, qui portent 1, 2 ou 3 substituants qui sont choisis parmi alkyle et les substituants mentionnés ci-dessus pour les groupes alkyle substitués, COOR^{a}, COO⁻M⁺, SO₃R^{a}, SO₃⁻M⁺, sulfonamide, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, alkylaminocarbonyle, dialkylaminocarbonyle, alkylcarbonylamino, halogène, nitro, acyle ou cyano, où
R^{a} signifie des radicaux à chaque fois identiques ou différents, choisis parmi hydrogène, alkyle, des groupes alkyle substitués, qui présentent 1, 2, 3, 4 ou 5 substituants, qui sont choisis parmi des groupes cycloalkyle, aryle, hétaryle, halogène, amino, alcoxycarbonyle, acyle, nitro, aminocarbonyle, alkylaminocarbonyle, dialkylaminocarbonyle, alkylcarbonylamino, carboxylate et sulfonate, cycloalkyle, des groupes cycloalkyle substitués, qui portent 1, 2, 3, 4 ou 5 substituants, qui sont choisis parmi alkyle et les substituants mentionnés ci-dessus pour les groupes alkyle, aryle, des groupes aryle substitués, qui portent 1, 2, 3, 4 ou 5 substituants, qui sont choisis parmi alkyle et les substituants mentionnés ci-dessus pour les groupes alkyle substitués, hétaryle ou des groupes hétérocycloaromatiques substitués, qui portent 1, 2 ou 3 substituants qui sont choisis parmi alkyle et les substituants mentionnés ci-dessus pour les groupes alkyle substitués.
E¹, E², E³ signifient à chaque fois des radicaux identiques ou différents, choisis parmi hydrogène, alkyle, des groupes alkyle substitués, qui présentent 1, 2, 3, 4 ou 5 substituants, qui sont choisis parmi des groupes cycloalkyle, aryle, hétaryle, halogène, amino, alcoxycarbonyle, acyle, nitro, aminocarbonyle, alkylaminocarbonyle, dialkylaminocarbonyle, alkylcarbonylamino, carboxylate et sulfonate, cycloalkyle, des groupes cycloalkyle substitués, qui portent 1, 2, 3, 4 ou 5 substituants, qui sont choisis parmi alkyle et les substituants mentionnés ci-dessus pour les groupes alkyle substitués, aryle, des groupes aryle substitués, qui portent 1, 2, 3, 4 ou 5 substituants, qui sont choisis parmi alkyle et les substituants mentionnés ci-dessus pour les groupes alkyle substitués, hétaryle ou des groupes hétérocycloaromatiques substitués, qui portent 1, 2 ou 3 substituants qui sont choisis parmi alkyle et les substituants mentionnés ci-dessus pour les groupes alkyle substitués,
R^{b} représente hydrogène, méthyle ou éthyle,
M⁺ représente un équivalent de cation,
A⁻ représente un équivalent d'anion et
y vaut un nombre entier de 1 à 250.

6. Procédé selon l'une quelconque des revendications 4 ou 5, où Z¹ et Z² représentent ensemble un groupe formant un pont avec deux ou trois atomes entre les liaisons adjacentes, qui sont choisis parmi des hétéroatomes le cas échéant substitués et des atomes de carbone hybridés en sp², le groupe formant le pont formant, avec le groupe NR¹-Y¹-Y², un système d'électrons π délocalisés.

7. Procédé selon l'une quelconque des revendications 4 à 6, où le composé de formule II est choisi parmi les composés de formule II.a à II.e 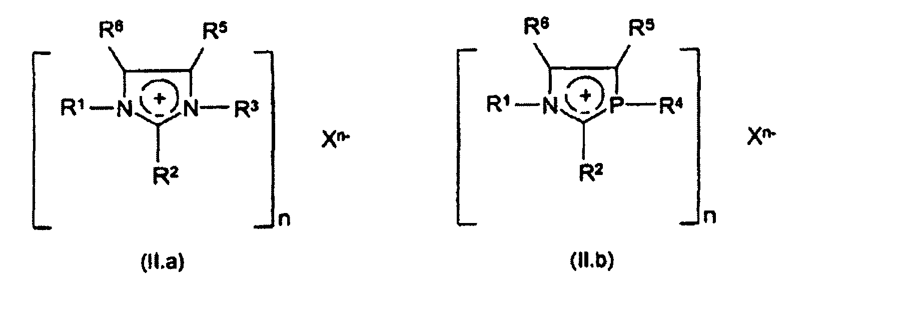 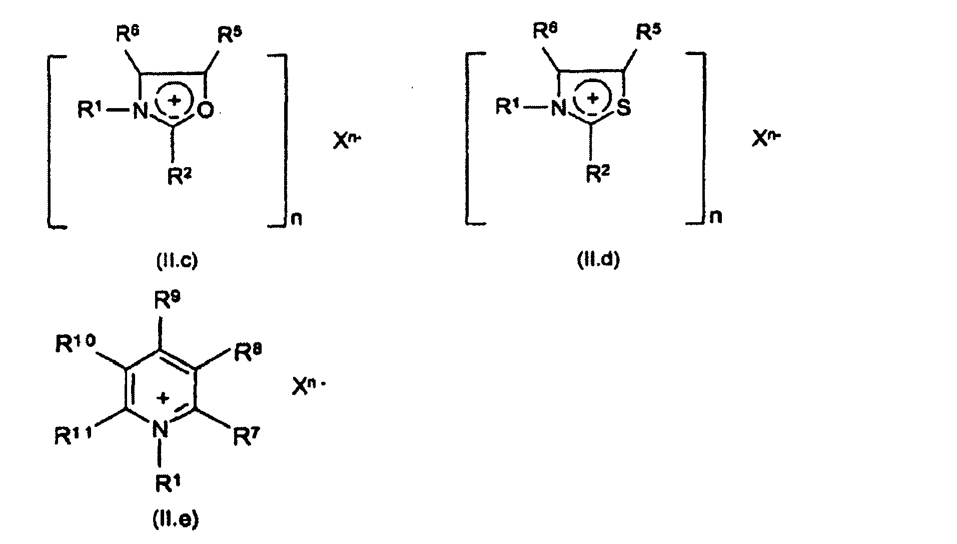 où
Xⁿ⁻ représente un anion qui est de préférence différent de Cl⁻, Br⁻, I⁻ et monoalkylsulfate, et
n vaut un nombre entier de 1 à 3,
R¹ représente alkyle en C₁-C₁₀, et
R², R³ ,R⁴, R⁵, R⁶, R⁷, R⁸, R⁹, R¹⁰ et R¹¹ représentent indépendamment l'un de l'autre, hydrogène, alkyle, des groupes alkyle substitués, qui présentent 1, 2, 3, 4 ou 5 substituants, qui sont choisis parmi des groupes cycloalkyle, aryle, hétaryle, halogène, amino, alcoxycarbonyle, acyle, nitro, aminocarbonyle, alkylaminocarbonyle, dialkylaminocarbonyle, alkylcarbonylamino, carboxylate et sulfonate, cycloalkyle, des groupes cycloalkyle substitués, qui portent 1, 2, 3, 4 ou 5 substituants, qui sont choisis parmi alkyle et les substituants mentionnés ci-dessus pour les groupes alkyle substitués, hétérocycloalkyle, des groupes hétérocycloaliphatiques substitués, qui portent 1, 2 ou 3 substituants, qui sont choisis parmi alkyle et les substituants mentionnés ci-dessus pour les groupes alkyle substitués, aryle, des groupes aryle substitués, qui portent 1, 2, 3, 4 ou 5 substituants, qui sont choisis parmi alkyle et les substituants mentionnés ci-dessus pour les groupes alkyle substitués, hétaryle, des groupes hétérocycloaromatiques substitués, qui portent 1, 2 ou 3 substituants qui sont choisis parmi alkyle et les substituants mentionnés ci-dessus pour les groupes alkyle substitués, COOR^{a}, CoO⁻M⁺, SO₃R^{a}, SO₃⁻M⁺, sulfonamide, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, alkylaminocarbonyle, dialkylaminocarbonyle, alkylcarbonylamino, halogène, nitro, acyle ou cyano, où
R^{a} signifie à chaque fois des radicaux identiques ou différents, choisis parmi hydrogène, alkyle, des groupes alkyle substitués, qui présentent 1, 2, 3, 4 ou 5 substituants, qui sont choisis parmi des groupes cycloalkyle, aryle, hétaryle, halogène, amino, alcoxycarbonyle, acyle, nitro, aminocarbonyle, alkylaminocarbonyle, dialkylaminocarbonyle, alkylcarbonylamino, carboxylate et sulfonate, cycloalkyle, des groupes cycloalkyle substitués, qui portent 1, 2, 3, 4 ou 5 substituants, qui sont choisis parmi alkyle et les substituants mentionnés ci-dessus pour les groupes alkyle substitués, aryle, des groupes aryle substitués, qui portent 1, 2, 3, 4 ou 5 substituants, qui sont choisis parmi alkyle et les substituants mentionnés ci-dessus pour les groupes alkyle substitués, hétaryle ou des groupes hétérocycloaromatiques substitués, qui portent 1, 2 ou 3 substituants qui sont choisis parmi alkyle et les substituants mentionnés ci-dessus pour les groupes alkyle substitués,
E¹, E², E³ signifient à chaque fois des radicaux identiques ou différents, choisis parmi hydrogène, alkyle, des groupes alkyle substitués, qui présentent 1, 2, 3, 4 ou 5 substituants, qui sont choisis parmi des groupes cycloalkyle, aryle, hétaryle, halogène, amino, alcoxycarbonyle, acyle, nitro, aminocarbonyle, alkylaminocarbonyle, dialkylaminocarbonyle, alkylcarbonylamino, carboxylate et sulfonate, cycloalkyle, des groupes cycloalkyle substitués, qui portent 1, 2, 3, 4 ou 5 substituants, qui sont choisis parmi alkyle et les substituants mentionnés ci-dessus pour les groupes alkyle substitués, aryle, des groupes aryle substitués, qui portent 1, 2, 3, 4 ou 5 substituants, qui sont choisis parmi alkyle et les substituants mentionnés ci-dessus pour les groupes alkyle substitués, hétaryle ou des groupes hétérocycloaromatiques substitués, qui portent 1, 2 ou 3 substituants qui sont choisis parmi alkyle et les substituants mentionnés ci-dessus pour les groupes alkyle substitués,
R^{b} représente hydrogène, méthyle ou éthyle,
M⁺ représente un équivalent de cation,
A⁻ représente un équivalent d'anion et
y vaut un nombre entier de 1 à 250.

8. Procédé selon l'une quelconque des revendications précédentes, où la transformation dans l'étape a) est réalisée à une température d'au moins 60°C, de préférence d'au moins 80°C, en particulier dans la plage de 100 à 220°C.

9. Procédé selon l'une quelconque des revendications précédentes, le rapport des quantités en mole du composé qui contient un atome d'azote lié doublement au dialkylsulfate étant d'au moins 2:1.

10. Procédé selon l'une quelconque des revendications précédentes, la transformation dans l'étape a) étant réalisée dans un solvant organique, dans l'eau ou dans un mélange de ceux-ci.

11. Procédé selon la revendication 10, où le solvant comprend au moins 30% en volume d'eau.

12. Procédé selon l'une quelconque des revendications précédentes, où la transformation dans l'étape a) est réalisée en présence d'un gaz inerte.

13. Procédé selon l'une quelconque des revendications précédentes, dans lequel le dialkylsulfate est choisi parmi le diméthylsulfate et le diéthylsulfate.

14. Procédé selon l'une quelconque des revendications précédentes, les étapes de procédé a) et b) étant réalisées en l'absence d'ions halogénure.

15. Procédé selon l'une quelconque des revendications précédentes, le remplacement de l'anion sulfate dans l'étape b) étant réalisé par transprotonation avec H₂SO₄, transformation avec un sel métallique, chromatographie par échange d'ions ou une combinaison de celles-ci.

16. Procédé selon la revendication 15, où la transformation avec le sel métallique est réalisée dans un solvant, à partir duquel un sulfate de métal formé à partir du métal du sel métallique et de l'anion sulfate se sépare par cristallisation.
